Europäisches Patentamt
European Patent Office
Office européen des brevets

(19) (11) Veröffentlichungsnummer: **0 269 839 A1**

(12) **EUROPÄISCHE PATENTANMELDUNG**

(21) Anmeldenummer: **87115569.3**

(22) Anmeldetag: **23.10.87**

(51) Int. Cl.⁴: **C07D 231/40**, C07D 401/04, A01N 43/56

(30) Priorität: **05.11.86 DE 3637710**

(43) Veröffentlichungstag der Anmeldung: **08.06.88 Patentblatt 88/23**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Stetter, Jörg, Dr.**
**Gellertweg 4**
**D-5600 Wuppertal 1(DE)**
Erfinder: **Schallner, Otto, Dr.**
**Noldeweg 22**
**D-4019 Monheim(DE)**
Erfinder: **Gehring Reinhold, Dr.**
**Dasnöckel 49**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Lürssen, Klaus, Dr.**
**August-Kierspel-Strasse 151**
**D-5060 Bergisch-Gladbach 2(DE)**
Erfinder: **Strang, Harry, Dr.**
**Unterdorfstrasse 6A**
**D-4000 Düsseldorf 31(DE)**

(54) **5-Acylamino-pyrazol-Derivate.**

(57) Die Erfindung betrifft neue 5-Acylamino-pyrazol-Derivate der Formel (I)

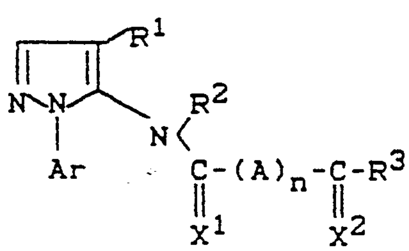

(I)

in welcher
$R^1$ für Wasserstoff. Halogen oder Nitro steht.
$R^2$ für Wasserstoff. Alkyl. Alkenyl. Alkinyl oder gegebenenfall substituiertes Cycloalkyl steht.
$R^3$ für Wasserstoff. Alkyl. für gegebenenfalls substituiertes Cycloalkyl-oder für gegebenenfalls substituiertes Aryl steht.

EP 0 269 839 A1

Ar für jeweils gegebenenfalls substituiertes Phenyl oder Pyridyl steht,
$X^1$ für Sauerstoff oder Schwefel steht,
A für eine geradkettige oder verzweigte, gegebenenfalls substituierte Alkylenbrücke steht,
n für die Zahlen 0 oder 1 steht und
$X^2$ für Sauerstoff, für einen Rest $=N-O-R^4$ oder für einen Rest

$$=N-N\left\langle\begin{matrix}R^5\\R^6\end{matrix}\right.$$

steht,
und $R^4$, $R^5$, und $R^6$ die in der Beschreibung angegebene Bedeutung haben,
mehrere Verfahren zu ihrer Herstellung sowie ihre Verwendung als Herbizide und Wachstumsregulatoren.

BAYER AKTIENGESELLSCHAFT 5090 Leverkusen, Bayerwerk
Konzernverwaltung RP
Patentabteilung KM/bo/c
(Ib)

# 5-Acylamino-pyrazol-Derivate

Die Erfindung betrifft neue 5-Acylamino-pyrazol-Derivate, mehrere Verfahren zu ihrer Herstellung sowie ihre Verwendung als Herbizide und Wachstumsregulatoren.

Es ist bereits bekannt, daß bestimmte 5-Acylamino-1-aryl-pyrazole, wie beispielsweise das 4-Cyano-5-propionamido-1-(2,4,6-trichlorphenyl)-pyrazol herbizide Eigenschaften besitzen (vgl. z.B. DE-OS 3 226 513).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsbereichen völlig zufriedenstellend.

Es wurden neue 5-Acylamino-pyrazol-Derivate der allgemeinen Formel (I),

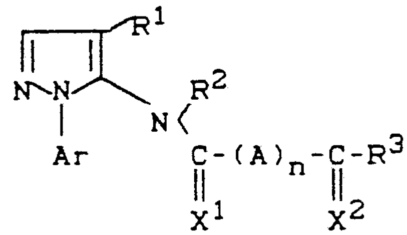

(I)

in welcher

$R^1$ für Wasserstoff, Halogen oder Nitro steht,

$R^2$ für Wasserstoff, Alkyl, Alkenyl, Alkinyl oder gegebenenfall substituiertes Cycloalkyl steht,

$R^3$ für Wasserstoff, Alkyl, für gegebenenfalls substituiertes Cycloalkyl oder für gegebenenfalls substituiertes Aryl steht,

Ar für jeweils gegebenenfalls substituiertes Phenyl oder Pyridyl steht,

$X^1$ für Sauerstoff oder Schwefel steht,

A für eine geradkettige oder verzweigte, gegebenenfalls substituierte Alkylenbrücke steht,

n für die Zahlen 0 oder 1 steht und

$X^2$ für Sauerstoff, für einen Rest $=N-O-R^4$ oder für einen Rest $=N-N\langle^{R^5}_{R^6}$ steht,

wobei

$R^4$ für Wasserstoff, Alkyl, Alkenyl, Alkinyl, Halogenalkyl, Halogenalkenyl, Alkoxyalkyl, Alkylthioalkyl, für gegebenenfalls substituiertes Aralkyl oder für gegebenenfalls substituiertes Aryl steht,

$R^5$ für Wasserstoff oder Alkyl steht und

$R^6$ für Wasserstoff, Alkyl, für gegebenenfalls substituiertes Aryl oder für einen Rest

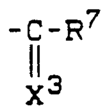 steht,

wobei

$X^3$ für Sauerstoff oder Schwefel steht und

$R^7$ für Wasserstoff, Alkyl, Alkoxy, Alkylamino oder Dialkylamino steht,

gefunden.

Weiterhin wurde gefunden, daß man die neuen 5-Acylamino-pyrazol-Derivate der Formel (I),

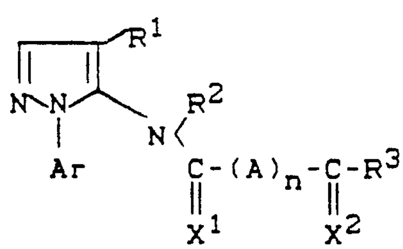 (I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff, Alkyl, für gegebenenfalls substituiertes Cycloalkyl oder für gegebenenfalls substituiertes Aryl steht,

Ar für jeweils gegebenenfalls substituiertes Phenyl oder Pyridyl steht,

$X^1$ für Sauerstoff oder Schwefel steht,

A für eine geradkettige oder verzweigte, gegebenenfalls substituierte Alkylenbrücke steht,

n für die Zahlen 0 oder 1 steht und

$X^2$ für Sauerstoff, für einen Rest $=N-O-R^4$ oder für einen Rest $=N-N\langle^{R^5}_{R^6}$ steht,

wobei

$R^4$ für Wasserstoff, Alkyl, Alkenyl, Alkinyl, Halogenalkyl, Halogenalkenyl, Alkoxyalkyl, Alkylthioalkyl, für gegebenenfalls substituiertes Aralkyl oder für gegebenenfalls substituiertes Aryl steht,

$R^5$ für Wasserstoff oder Alkyl steht und

$R^6$ für Wasserstoff, Alkyl, für gegebenenfalls substituiertes Aryl oder für einen Rest

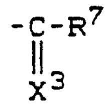 steht,

wobei

$X^3$ für Sauerstoff oder Schwefel steht und

$R^7$ für Wasserstoff, Alkyl, Alkoxy, Alkylamino oder Dialkylamino steht,

mit Hilfe der im folgenden beschriebenen Verfahren erhält:

a) Man erhält 5-Acylamino-pyrazol-Derivate der Formel (Ia),

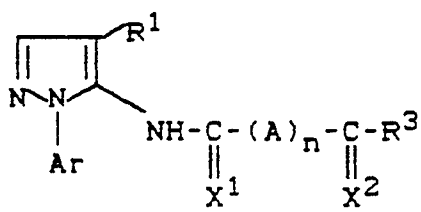

(Ia)

in welcher

$R^1$, $R^3$, $X^1$, $X^2$, A, Ar und der Index n die oben angegebene Bedeutung haben,

wenn man 5-Amino-1-aryl-pyrazole der Formel (II),

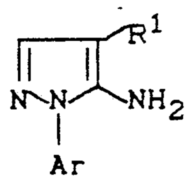

(II)

in welcher

$R^1$ und Ar die oben angegebene Bedeutung haben,

mit Acylierungsmitteln der Formel (III),

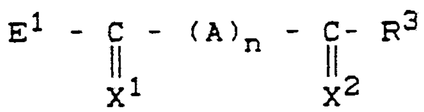

(III)

in welcher
$X^1$, $X^2$, $R^3$, A und der Index n die oben angegebene Bedeutung haben und

$E^1$ für eine elektronenanziehende Abgangsgruppe steht,

gegebenenfalls in Gegenwart eines Verdünnungsmittels, gegebenenfalls in Gegenwart eines Säurebindemittels sowie gegebenenfalls in Gegenwart eines Katalysators umsetzt;

b) man erhält 5-Acylamino-pyrazol-Derivate der Formel (Ib),

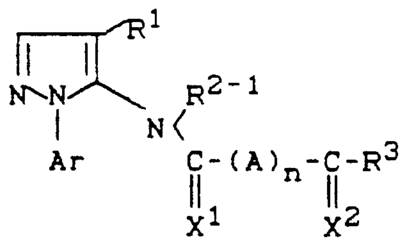

(Ib)

in welcher

$R^1$, $R^3$, $X^1$, $X^2$, A , der Index n und Ar die oben angegebene Bedeutung haben und

$R^{2-1}$ für Alkyl, Alkenyl, Alkinyl oder gegebenenfalls substituiertes Cycloalkyl steht,

wenn man die nach Verfahren (a) erhältlichen 5-Acylamino-pyrazol-Derivate der Formel (Ia),

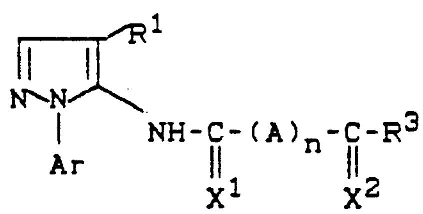

(Ia)

in welcher

$R^1$, $R^3$, $X^1$, $X^2$, A, Ar und der Index n die oben angegebene Bedeutung haben,

mit Alkylierungsmitteln der Formel (IV),

$$E^2 - R^{2-1} \qquad (IV)$$

in welcher

$R^{2-1}$ die oben angegebene Bedeutung hat und

$E^2$ für Halogen, für gegebenenfalls substituiertes Alkoxysulfonyloxy oder für gegebenenfalls substituiertes Arylsulfonyloxy steht,

gegebenenfalls in Gegenwart eines Verdünnungsmittels und gegebenenfalls in Gegenwart eines Säurebindemittels sowie gegebenenfalls in Gegenwart eines Katalysators umsetzt;

c) man erhält 5-Acylamino-pyrazol-Derivate der Formel (Ic),

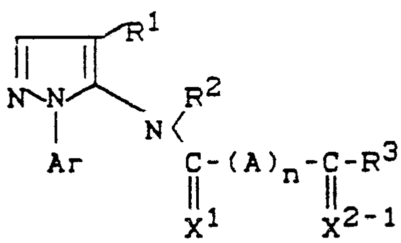

(Ic)

in welcher

$R^1$, $R^2$, $R^3$, $X^1$, A, Ar und der Index n die oben angegebene Bedeutung haben und

$X^{2-1}$ für einen Rest $=N-O-R^4$ oder für einen Rest

$=N-N{<}^{R^5}_{R^6}$ steht,

wobei

$R^4$, $R^5$ und $R^6$ die oben angegebene Bedeutung haben,

wenn man die nach Verfahren (a) oder (b) erhältlichen 5-Acylamino-pyrazol-Derivate der Formel (Ie),

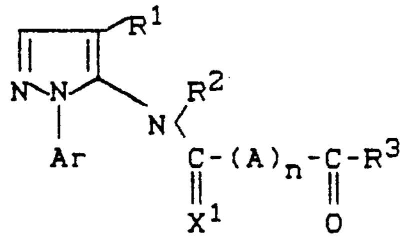

(Ie)

in welcher

$R^1$, $R^2$, $R^3$, $X^1$, A, Ar und der Index n die oben angegebene Bedeutung haben,

mit primären Aminoverbindungen der Formel (V),

$$H_2N - Y \quad (V)$$

in welcher

Y für einen Rest $-OR^4$ oder für einen Rest $-N\langle^{R^5}_{R^6}$ steht, wobei $R^4$, und $R^5$ und $R^6$ die oben angegebene Bedeutung haben,

gegebenenfalls in Gegenwart eines Verdünnungsmittels und

gegebenenfalls in Gegenwart eines Reaktionshilfsmittels umsetzt;

d) man erhält 5-Acylamino-pyrazol-Derivate der Formel (Id),

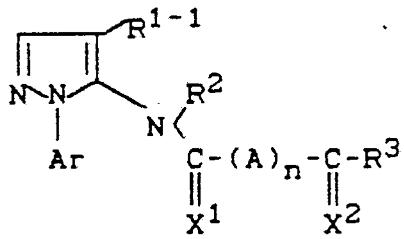

(Id)

in welcher

$R^2$, $R^3$, $X^1$, $X^2$, A, Ar und der Index n die oben angegebene Bedeutung haben und

$R^{1-1}$ für Halogen oder Nitro steht,

wenn man die mit Hilfe der Verfahren (a), (b) oder (c) erhältlichen 5-Acylamino-pyrazol-Derivate der Formel (If),

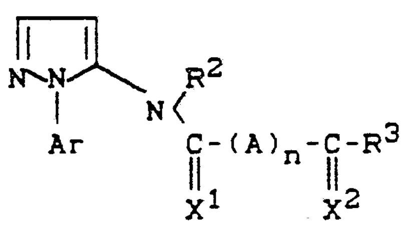

(If)

in welcher

$R^2$, $R^3$, $X^1$, $X^2$, A, Ar und der Index n die oben angegebene Bedeutung haben,

mit Halogenierungs- oder Nitrierungsmitteln der Formel (VI),

$$R^{1-1} - E^3 \quad (VI)$$

in welcher

$R^{1-1}$ die oben angegebene Bedeutung hat und

$E^3$ für eine elektronenanziehende Abgangsgruppe steht,

gegebenenfalls in Gegenwart eines Verdünnungsmittels und gegebenenfalls in Gegenwart eines Katalysators oder Reaktionshilfsmittels umsetzt.

Schließlich wurde gefunden, daß die neuen 5-Acylamino-pyrazol-Derivate der allgemeinen Formel (I) herbizide, insbesondere auch selektiv-herbizide und wachstumsregulierende Eigenschaften besitzen.

Überraschenderweise zeigen die erfindungsgemäßen 5-Acylamino-pyrazol-Derivate der allgemeinen Formel (I) eine erheblich bessere allgemein-herbizide Wirksamkeit gegen schwer bekämpfbare Problemunkräuter und gleichzeitig eine deutlich verbesserte Verträglichkeit gegenüber wichtigen Kulturpflanzen im Vergleich zu den aus dem Stand der Technik bekannten 5-Acylamino-1-aryl-pyrazolen, wie beispielsweise dem 4-Cyano-5-propionamido-1-(2,4,6-trichlorphenyl)-pyrazol, welche chemisch und wirkungsmäßig naheliegende Verbindungen sind.

Die erfindungsgemäßen 5-Acylamino-pyrazol-Derivate sind durch die Formel (I) allgemein definiert. Bevorzugt sind Verbindungen der Formel (I), bei welchen

$R^1$ für Wasserstoff, Nitro, Fluor, Chlor, Brom oder Iod steht,

$R^2$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 3 bis 8 Kohlenstoffatomen sowie für gegebenenfalls einfach oder mehrfach gleich oder verschieden durch $C_1$-$C_4$-Alkyl substituiertes Cycloalkyl mit 3 bis 8 Kohlenstoffatomen steht,

$R^3$ für Wasserstoff, für geradkettiges oder verzweigtes Alkyl mit 1 bis 8 Kohlenstoffatomen, für gegebenenfalls einfach oder mehrfach, gleich oder verschieden durch $C_1$-$C_4$-Alkyl substituiertes Cycloalkyl mit 3 bis 8 Kohlenstoffatomen oder für gegebenenfalls einfach oder mehrfach, gleich oder verschieden substituiertes Aryl mit 6 bis 10 Kohlenstoffatomen steht, wobei als Arylsubstituenten in Frage kommen: Halogen, Cyano, Nitro sowie jeweils geradkettiges oder verzweigtes Alkyl oder Alkoxy mit jeweils 1 bis 4 Kohlenstoffatomen,

Ar für jeweils gegebenenfalls einfach oder mehrfach gleich oder verschieden substituiertes Phenyl, 2-Pyridyl, 3-Pyridyl oder 4-Pyridyl steht, wobei als

Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m - R^8$,

wobei

$R^8$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für eine Zahl 0, 1 oder 2 steht,

$X^1$ für Sauerstoff oder Schwefel steht,

A für eine geradkettige oder verzweigte Alkylenbrücke mit 1 bis 6 Kohlenstoffatomen steht, welche gegebenenfalls einfach oder mehrfach, gleich oder verschieden durch Phenyl, das gegebenenfalls einfach oder mehrfach, gleich oder verschieden durch Halogen oder $C_1$-$C_4$-Alkyl substituiert ist, oder durch Halogen substituiert ist,

n für eine Zahl 0 oder 1 steht und

$X^2$ für Sauerstoff, für einen Rest $=N-O-R^4$ oder für einen Rest $=N-N\langle\begin{smallmatrix}R^5\\R^6\end{smallmatrix}$ steht, wobei

$R^4$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 8 Kohlenstoffatomen, für jeweils geradkettiges oder verzweigtes Halogenalkyl mit 1 bis 8 Kohlenstoffatomen oder Halogenalkenyl mit 3 bis 8 Kohlenstoffatomen und jeweils 1 bis 9 gleichen oder verschiedenene Halogenatomen, für jeweils geradkettiges oder verzweigtes Alkoxyalkyl oder Alkylthioalkyl mit jeweils 1 bis 4 Kohlenstoffatomen in den einzelnen Alkylteilen oder für jeweils im Arylteil einfach oder mehrfach, gleich oder verschieden substituiertes Aralkyl mit 1 bis 4 Kohlenstoffatomen im Alkylteil oder Aryl mit jeweils 6 bis 10 Kohlenstoffatomen im Arylteil steht, wobei als Arylsubstituenten jeweils infrage kommen: Halogen, Cyano, Nitro, sowie jeweils geradkettiges oder verzweigtes Alkyl oder Alkoxy mit jeweils 1 bis 4 Kohlenstoffatomen,

$R^5$ für Wasserstoff oder für geradkettiges oder verzweigtes Alkyl mit 1 bis 6 Kohlenstoffatomen steht und

$R^6$ für Wasserstoff, für geradkettiges oder verzweigtes Alkyl mit 1 bis 6 Kohlenstoffatomen, für gegebenenfalls einfach oder mehrfach, gleich oder verschieden substituiertes Aryl mit 6 bis 10 Kohlenstoffatomen oder für einen Rest $-\underset{\underset{X^3}{\|}}{C}-R^7$

steht,

wobei als Arylsubstituenten die bei $R^4$ genannten infrage kommen,

$X^3$ für Sauerstoff und Schwefel steht und

$R^7$ für Wasserstoff oder für jeweils geradkettiges oder verzweigtes Alkyl, Alkoxy, Alkylamino oder Dialkylamino mit jeweils 1 bis 8 Kohlenstoffatomen in den einzelnen Alkylteilen steht.

Besonders bevorzugt sind 5-Acylamino-pyrazol-Derivate der Formel (I), bei welchen

$R^1$ für Wasserstoff, Nitro, Chlor oder Brom steht,

$R^2$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 6 Kohlenstoffatomen sowie für gegebenenfalls einfach bis dreifach, gleich oder verschieden durch Methyl, Ethyl oder Isopropyl substituiertes Cycloalkyl mit 3 bis 6 Kohlenstoffatomen steht,

$R^3$ für Wasserstoff, für geradkettiges oder verzweigtes Alkyl mit 1 bis 6 Kohlenstoffatomen, für gegebenenfalls einfach bis dreifach, gleich oder verschieden durch Methyl, Ethyl oder Isopropyl substituiertes Cycloalkyl mit 3 bis 6 Kohlenstoffatomen oder für gegebenenfalls ein- bis fünffach, gleich oder verschieden substituiertes Phenyl steht, wobei als Phenylsubstituenten infrage kommen: Fluor, Chlor, Brom, Iod, Cyano, Nitro, Methyl, Ethyl, n- oder i-Propyl, n-, i-, s- oder t-Butyl, Methoxy oder Ethoxy,

Ar für gegebenenfalls ein- bis fünffach, gleich oder verschieden substituiertes Phenyl oder für jeweils gegebenenfalls ein- bis vierfach, gleich oder verschieden substituiertes 2-Pyridyl, 3-Pyridyl oder 4-Pyridyl steht, wobei als Phenyl- und Pyridyl-Substituenten jeweils infrage kommen: Cyano, Nitro, Fluor, Chlor, Brom, Iod, Methyl, Ethyl, n- oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^8$,

wobei

$R^8$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und

m für eine Zahl 0, 1 oder 2 steht,

$X^1$ für Sauerstoff oder Schwefel steht,

A für ein Brückenglied der Formel $-CH_2-$;

$-CH_2-CH_2-$; $-\underset{\underset{CH_3}{|}}{C}H-$; $-CCl_2-CH_2-$; $-CHCl-CH_2-$,

$-\underset{\underset{CH_3}{|}}{C}H-CH_2-$; $-CH_2-CH_2-CH_2-$; $-CH_2-CH_2-CH_2-CH_2-$

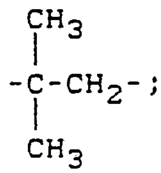 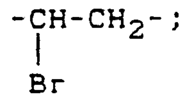 $-CBr_2-CH_2-$; $-C(CH_3)_2-$;

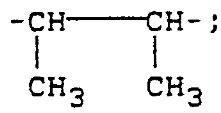 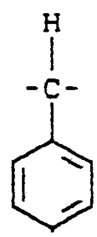 oder 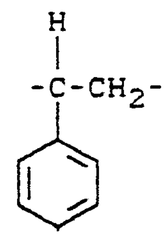 steht;

n für eine Zahl 0 oder 1 steht und

$X^2$ für Sauerstoff, für einen Rest $=N-O-R^4$ oder für einen Rest

$$=N-N\langle^{R^5}_{R^6}$$ steht,

wobei

$R^4$ für Wasserstoff, für geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen, für jeweils geradkettiges oder verzweigtes Halogenalkyl mit 1 bis 6 Kohlenstoffatomen oder Halogenalkenyl mit 3 bis 6 Kohlenstoffatomen und jeweils 1 bis 5 gleichen oder verschiedenen Halogenatomen

(insbesondere Fluor, Chlor oder Brom), für jeweils geradkettiges oder verzweigtes Alkoxyalkyl oder Alkylthioalkyl mit jeweils 1 bis 3 Kohlenstoffatomen in den einzelnen Alkylteilen oder für jeweils im Phenylteil ein- bis fünffach, gleich oder verschieden substituiertes Benzyl, Phenylethyl oder Phenyl steht, wobei als Phenylsubstituenten jeweils infrage kommen: Fluor, Chlor, Brom, Iod, Cyano, Nitro, Methyl, Ethyl, n- oder i-Propyl, n-, i-, s- oder t-Butyl, Methoxy oder Ethoxy,

$R^5$ für Wasserstoff oder für geradkettiges oder verzweigtes Alkyl mit 1 bis 4 Kohlenstoffatomen steht und

$R^6$ für Wasserstoff, für geradkettiges oder verzweigtes Alkyl mit 1 bis 4 Kohlenstoffatomen, für gegebenenfalls ein- bis fünffach, gleich oder verschieden substituiertes Phenyl oder für einen Rest

$$-\underset{\underset{X^3}{\|}}{C}-R^7$$ steht,

wobei als Phenylsubstituenten die bei $R^4$ genannten infrage kommen,

$X^3$ für Sauerstoff oder Schwefel steht und

$R^7$ für Wasserstoff, für geradkettiges oder verzweigtes Alkyl mit 1 bis 6 Kohlenstoffatomen oder für jeweils geradkettiges oder verzweigtes Alkoxy, Alkylamino oder Dialkylamino mit jeweils 1 bis 4 Kohlenstoffatomen in den einzelnen Alkylteilen steht.

Ganz besonders bevorzugt sind 5-Acylamino-pyrazol-Derivate der Formel (I), bei welchen

$R^1$ für Wasserstoff oder Nitro steht,

$R^2$ für Wasserstoff, Methyl, Ethyl, Allyl, Propargyl oder Cyclohexyl steht,

$R^3$ für Wasserstoff, Methyl, Ethyl, n- oder i-Propyl, n-, i-, s- oder t-Butyl, für Cyclopropyl, Cyclobutyl, Cyclopentyl, Cyclohexyl, Methylcyclopropyl oder Dimethylcyclopropyl oder für gegebenenfalls ein- bis fünffach gleich oder verschieden substituiertes Phenyl steht, wobei als Phenylsubstituenten infrage kommen: Cyano, Nitro, Fluor, Chlor, Brom, Methyl, Ethyl, n- oder i-Propyl, n-, i-, s- oder t-Butyl, Methoxy oder Ethoxy,

Ar für gegebenenfalls ein- bis fünffach, gleich oder verschieden substituiertes Phenyl oder für jeweils gegebenenfalls ein- bis vierfach, gleich oder verschieden substituiertes 2-Pyridyl, 3-Pyridyl oder

4-Pyridyl steht, wobei als Phenyl- und Pyridyl-Substituenten jeweils in Frage kommen: Cyano, Nitro, Fluor, Chlor, Brom, Iod, Methyl, Ethyl, n- oder i-Propyl, n-, i-, s-oder t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^8$,

wobei

$R^8$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0, 1 oder 2 steht sowie

$X^1$ für Sauerstoff oder Schwefel steht,

A für ein Brückenglied der Formel $-CH_2-$; $-CH_2-CH_2-$;

$-CH_2-CH_2-CH_2-$; 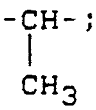; 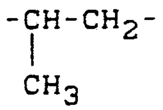; 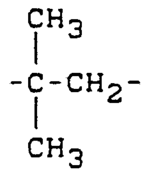; 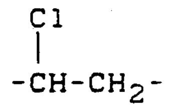;

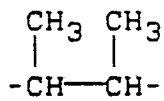; 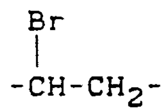, $-CCl_2-CH_2$; $-C(CH_3)_2-$ oder

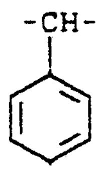 steht,

n für eine Zahl 0 oder 1 steht und

$X^2$ für Sauerstoff, für einen Rest $=N-OR^4$ oder für einen Rest

$$=N-N\langle{}^{R^5}_{R^6}$$ steht, wobei

$R^4$ für Wasserstoff, Methyl, Ethyl, n- oder i-Propyl, n-, i-, s- oder t-Butyl, für Allyl, Butenyl, Pentenyl, für Propargyl, Butinyl, Chlorethyl, Trifluorethyl, Bromethyl, Chlorallyl, Bromallyl, Dichlorpropenyl, Chlorbutenyl, Methoxymethyl, Methoxyethyl, Ethoxymethyl, Ethoxyethyl, Methylthiomethyl, Methylthioethyl, Ethylthiomethyl, Ethylthioethyl oder für jeweils im Phenylteil ein- bis fünffach, gleich oder verschieden substituiertes

Benzyl, Phenylethyl oder Phenyl steht, wobei als Phenylsubstituenten jeweils infrage kommen: Fluor, Chlor, Brom, Cyano, Nitro, Methyl, Ethyl, n- oder i-Propyl, n-, i-, s- oder t-Butyl, Methoxy oder Ethoxy,

$R^5$ für Wasserstoff, Methyl, Ethyl, n- oder i-Propyl, n-, i, s- oder t-Butyl steht und

$R^6$ für Wasserstoff, Methyl, Ethyl, n- oder i-Propyl, n-, i-, s- oder t-Butyl, für gegebenenfalls ein- bis fünffach, gleich oder verschieden substituiertes Phenyl oder für einen Rest

$$-\underset{\underset{X^3}{\|}}{C}-R^7$$ steht,

wobei als Phenylsubstituenten die bei $R^4$ genannten infrage kommen,

$X^3$ für Sauerstoff oder Schwefel steht und

$R^7$ für Wasserstoff, Methyl, Ethyl, n- oder i-Propyl, n-, i-, s- oder t- Butyl, Methoxy, Ethoxy, n- oder i-Propoxy, n-, i-, s- oder t-Butoxy, Methylamino, Ethylamino, Dimethylamino, Diethylamino, Propylamino oder Dipropylamino steht.

Im einzelnen seien außer den bei den Herstellungsbeispielen genannten Verbindungen die folgenden 5-Acylamino-pyrazol-Derivate der allgemeinen Formel (I) genannt:

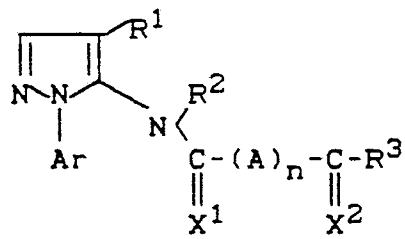 (I)

Tabelle 1

| $R^1$ | $R^2$ | $X^1$ | n | A | 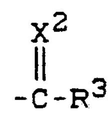 | Ar |
|---|---|---|---|---|---|---|
| H | H | O | 0 | - | 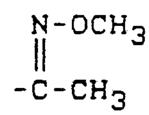 | 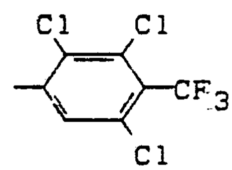 |
| H | H | O | 0 | - | 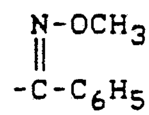 | 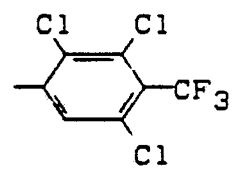 |
| H | H | O | 0 | - | 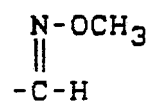 | 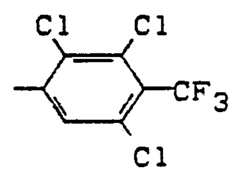 |
| H | H | O | 0 | - | 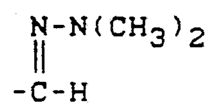 | 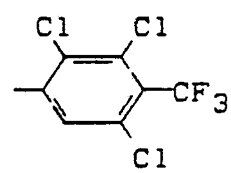 |

Tabelle 1 - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | O | - | $N-OC_2H_5$<br>‖<br>$-C-H$ | 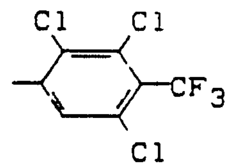 |
| $NO_2$ | H | O | O | - | $N-NH-CH_3$<br>‖<br>$-C-CH_3$ | 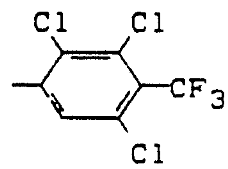 |
| $NO_2$ | H | O | O | - | O<br>‖<br>$N-NH-C-NH-CH_3$<br>‖<br>$-C-CH_3$ | 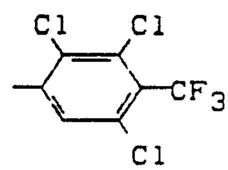 |
| $NO_2$ | H | O | O | - | $N-NH_2$<br>‖<br>$-C-C_6H_5$ | 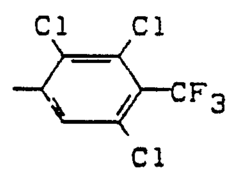 |
| $NO_2$ | H | O | O | - | $N-OC_2H_5$<br>‖<br>$-C-H$ | 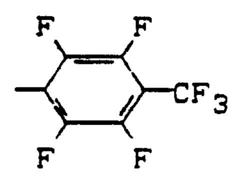 |
| $NO_2$ | H | O | O | - | $N-OCH_3$<br>‖<br>$-C-C_6H_5$ | 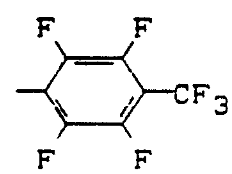 |

Tabelle 1 - Fortsetzung

| $R^1$ | $R^2$ | $X^1$ | n | A | $-\overset{X^2}{\overset{\|}{C}}-R^3$ | Ar |
|---|---|---|---|---|---|---|
| $NO_2$ | H | O | O | - | $-\overset{N-OCH_3}{\overset{\|}{C}}-CH_3$ | 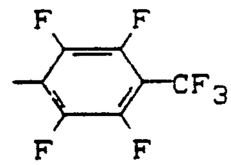 |
| $NO_2$ | H | O | O | - | $-\overset{N-OC_2H_5}{\overset{\|}{C}}-C_2H_5$ | 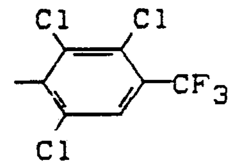 |
| $NO_2$ | H | O | O | - | $-\overset{O}{\overset{\|}{C}}-CH_3$ | 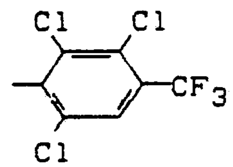 |
| $NO_2$ | H | O | O | - | $-\overset{O}{\overset{\|}{C}}-C_2H_5$ | 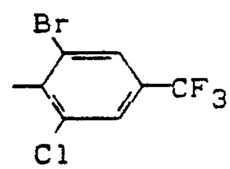 |
| $NO_2$ | H | O | O | - | $-\overset{O}{\overset{\|}{C}}-C_6H_5$ | 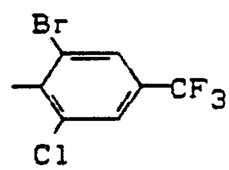 |
| $NO_2$ | H | O | O | - | 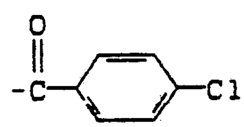 | 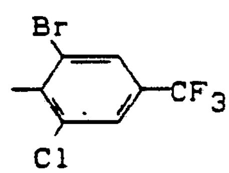 |
| $NO_2$ | H | O | O | - | 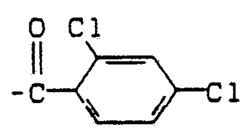 | 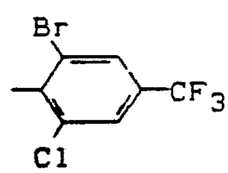 |

Tabelle 1 - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | O | - | $N-OCH_3$<br>‖<br>$-C-C_6H_5$ | 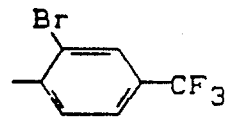 |
| $NO_2$ | H | O | O | - | $N-OCH_3$<br>‖<br>$-C-H$ | 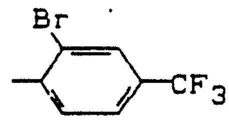 |
| $NO_2$ | H | O | O | - | $N-OCH_3$<br>‖<br>$-C-CH_3$ | 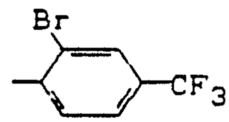 |
| $NO_2$ | H | O | O | - | O<br>‖<br>$-C-H$ | 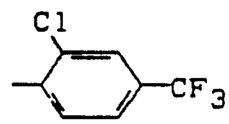 |
| $NO_2$ | H | O | O | - | $N-N(CH_3)_2$<br>‖<br>$-C-H$ | 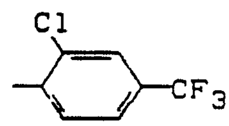 |
| $NO_2$ | H | O | O | - | O<br>‖<br>$-C-CH_3$ | 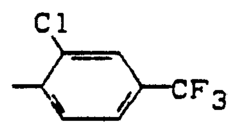 |
| $NO_2$ | H | O | O | - | O<br>‖<br>$-C-CH_3$ | 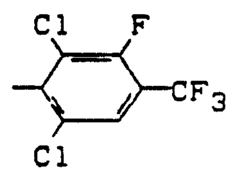 |
| $NO_2$ | H | O | O | - | $N-OCH_3$<br>‖<br>$-C-H$ | 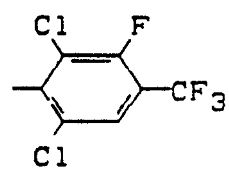 |

Tabelle 1 - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | O | - | $N-OCH_3$<br>‖<br>$-C-CH_3$ | 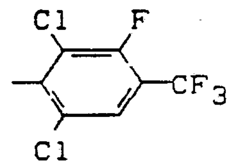 |
| $NO_2$ | H | O | O | - | O<br>‖<br>-C-H | 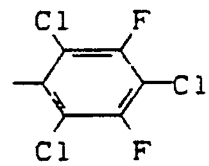 |
| $NO_2$ | H | O | O | - | $N-OCH_3$<br>‖<br>-C-H | 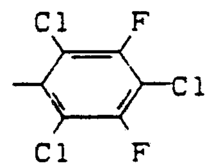 |
| $NO_2$ | H | O | O | - | $N-OCH_3$<br>‖<br>-C-H | 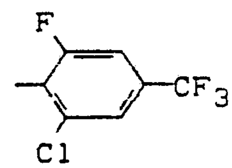 |
| $NO_2$ | H | O | O | - | $N-OCH_3$<br>‖<br>-C-H | 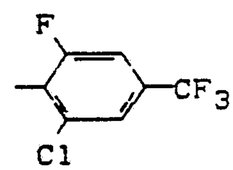 |
| $NO_2$ | H | O | 1 | $-CH_2-$ | $N-OCH_3$<br>‖<br>-C-H | 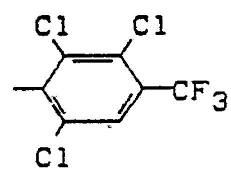 |
| $NO_2$ | H | O | 1 | $CH_3$<br>\|<br>-CH- | O<br>‖<br>-C-H | 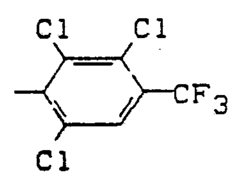 |

Tabelle 1 - Fortsetzung

| $R^1$ | $R^2$ | $X^1$ | n | A | $-C(=X^2)-R^3$ | Ar |
|---|---|---|---|---|---|---|
| $NO_2$ | H | O | 1 | $-CH_2-$ | $-C(=N-OC_2H_5)-H$ | 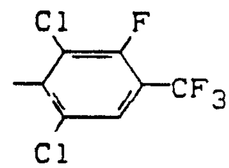 |
| $NO_2$ | H | O | 1 | $-CH(CH_3)-$ | $-C(=N-OCH_3)-C_2H_5$ | 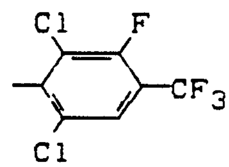 |
| $NO_2$ | H | O | 1 | $-CH_2-$ | $-C(=N-OH)-CH_3$ | 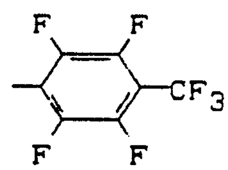 |
| $NO_2$ | H | O | 1 | $-CH(CH_3)-$ | $-C(=N-OCH_3)-H$ | 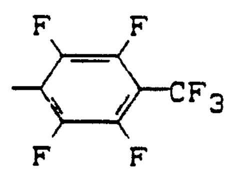 |
| $NO_2$ | H | O | 1 | $-CH_2-$ | $-C(=N-OH)-C_6H_5$ | 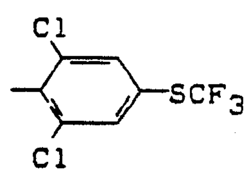 |
| $NO_2$ | H | O | 1 | $-CH_2-$ | $-C(=O)-H$ | 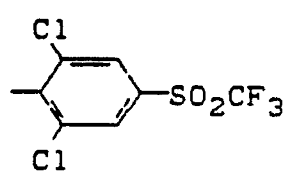 |
| $NO_2$ | H | O | 1 | $-CH_2-$ | $-C(=O)-CH_3$ | 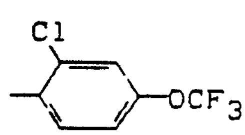 |

Tabelle 1 - Fortsetzung

| $R^1$ | $R^2$ | $X^1$ | n | A | 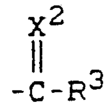 | Ar |
|---|---|---|---|---|---|---|
| $NO_2$ | H | O | 1 | $-CH_2-$ | 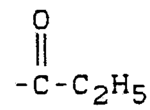 | 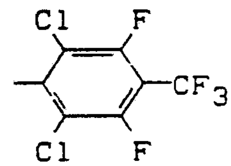 |
| $NO_2$ | H | O | 1 | $-CH_2-$ | 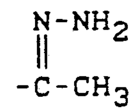 | 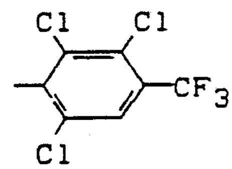 |
| $NO_2$ | H | O | 1 | 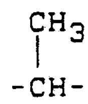 | 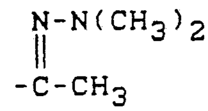 | 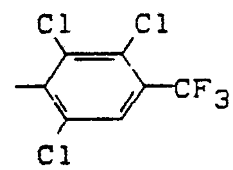 |
| $NO_2$ | H | O | 1 | 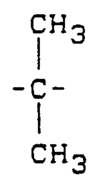 | 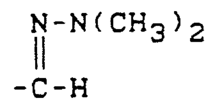 | 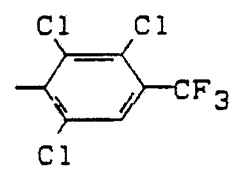 |
| $NO_2$ | H | O | 1 | $-CH_2-$ | 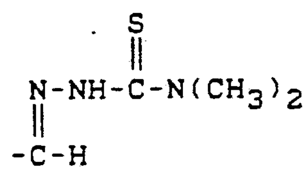 | 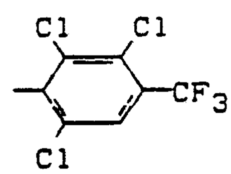 |
| $NO_2$ | H | O | 1 | 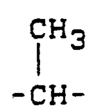 | 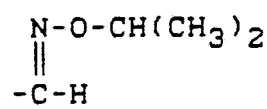 | 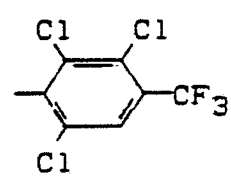 |

Tabelle 1 - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | 1 | $-CH_2-$ | O<br>‖<br>$-C-CH(CH_3)_2$ | 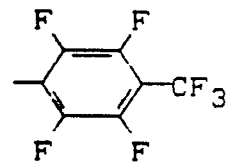 |
| $NO_2$ | H | O | 1 | $-CH_2-$ | O<br>‖<br>$-C-C_2H_5$ | 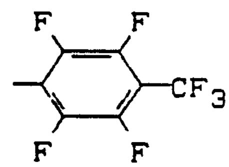 |
| $NO_2$ | H | O | 1 | $-CH_2-$ | $N-O-CH(CH_3)_2$<br>‖<br>$-C-CH_3$ | 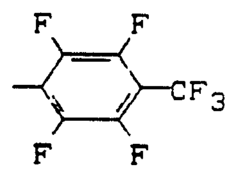 |
| $NO_2$ | H | O | 1 | $CH_3$<br>\|<br>-CH- | N-OH<br>‖<br>$-C-CH_3$ | 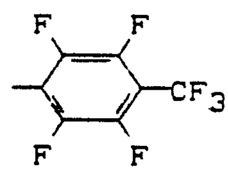 |
| $NO_2$ | H | O | 1 | $-CH_2-$ | N-OH<br>‖<br>$-C-CH_3$ | 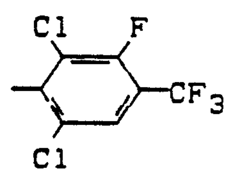 |
| $NO_2$ | H | O | 1 | $CH_3$<br>\|<br>-CH- | 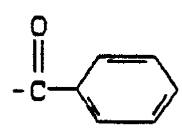 | 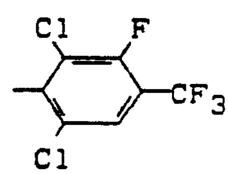 |
| $NO_2$ | H | O | 1 | $-CH_2CH_2-$ | 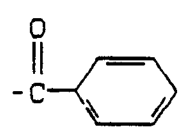 | 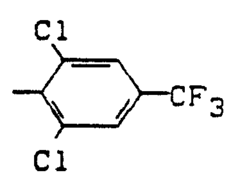 |

Tabelle 1 - Fortsetzung

| $R^1$ | $R^2$ | $X^1$ | n | A | 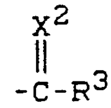 | Ar |
|---|---|---|---|---|---|---|
| $NO_2$ | H | O | 1 | $-CH_2CH_2-$ | 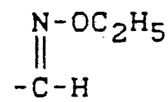 | 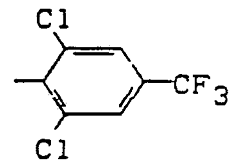 |
| $NO_2$ | H | O | 1 | 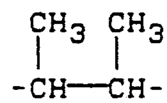 | 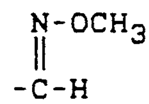 | 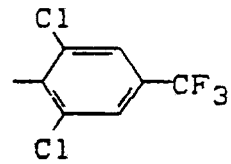 |
| $NO_2$ | H | O | 1 | 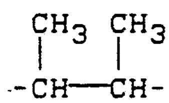 | 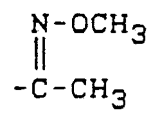 | 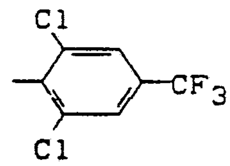 |
| $NO_2$ | H | O | 1 | 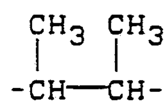 | 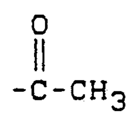 | 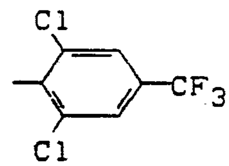 |
| $NO_2$ | H | O | 1 | $-CH_2CH_2-$ | 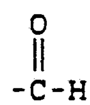 | 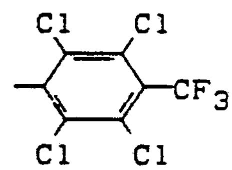 |
| $NO_2$ | H | O | 1 | $-CH_2CH_2-$ | 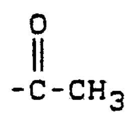 | 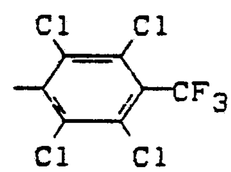 |
| $NO_2$ | H | O | 1 | $-CH_2CH_2-$ | 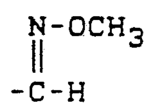 | 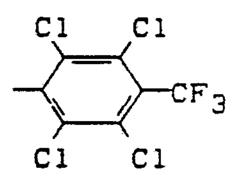 |

Tabelle 1 - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | 1 | $-CH_2CH_2-$ | $N-OC_2H_5$<br>‖<br>$-C-H$ | 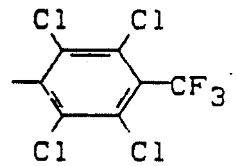 |
| $NO_2$ | H | O | 1 | $-CH_2-$ | O<br>‖<br>$-C-CH_3$ | 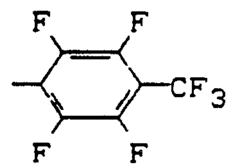 |
| $NO_2$ | H | O | 1 | $-CH_2-$ | 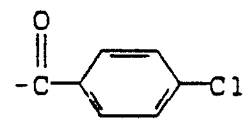 | 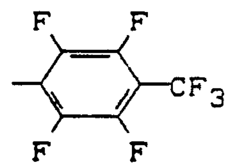 |
| $NO_2$ | H | O | 1 | $-CH_2-$ | $N-O-CH(CH_3)_2$<br>‖<br>$-C-H$ | 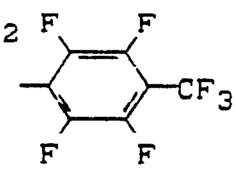 |
| $NO_2$ | H | O | 0 | - | $N-NH_2$<br>‖<br>$-C-CH_3$ | 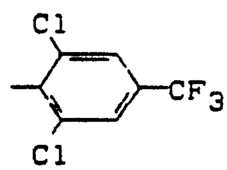 |
| $NO_2$ | H | O | 0 | - | $N-N(CH_3)_2$<br>‖<br>$-C-H$ | 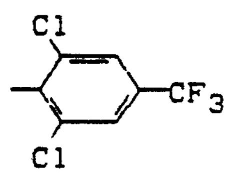 |
| $NO_2$ | H | O | 1 | $-CH_2-$ | $N-N(CH_3)_2$<br>‖<br>$-C-CH_3$ | 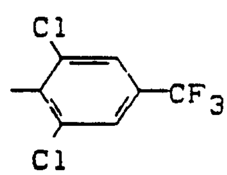 |

Tabelle 1 - Fortsetzung

| $R^1$ | $R^2$ | $X^1$ | n | A | $-\overset{X^2}{\overset{\|}{C}}-R^3$ | Ar |
|---|---|---|---|---|---|---|
| $NO_2$ | H | O | 1 | $-CH_2-$ | $-\overset{O}{\overset{\|}{C}}-C_2H_5$ | 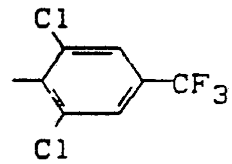 |
| $NO_2$ | H | O | 1 | $-\overset{CH_3}{\overset{\|}{C}}H-$ | $-\overset{N-OH}{\overset{\|}{C}}-C_2H_5$ | 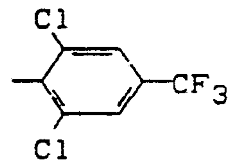 |
| $NO_2$ | H | O | 1 | $-CH_2CH_2-$ | $-\overset{N-N(CH_3)_2}{\overset{\|}{C}}-C_2H_5$ | 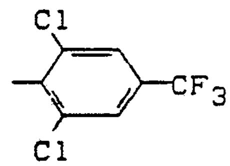 |
| $NO_2$ | H | O | 1 | $-CH_2CH_2-$ | $-\overset{O}{\overset{\|}{C}}-CH(CH_3)_2$ | 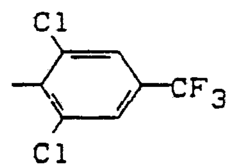 |
| $NO_2$ | H | O | 1 | $-\overset{CH_3}{\overset{\|}{C}}H-\overset{CH_3}{\overset{\|}{C}}H-$ | $-\overset{O}{\overset{\|}{C}}-H$ | 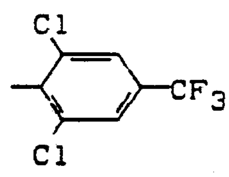 |
| $NO_2$ | H | O | 1 | $-CH_2CH_2-$ | $-\overset{N-N(CH_3)_2}{\overset{\|}{C}}-CH_3$ | 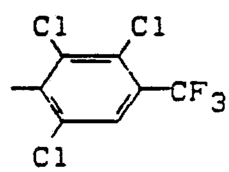 |
| $NO_2$ | H | O | 1 | $-CH_2CH_2-$ | $-\overset{O}{\overset{\|}{C}}-CH(CH_3)_2$ | 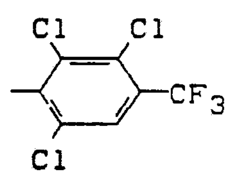 |

<u>Tabelle 1</u> - Fortsetzung

| $R^1$ | $R^2$ | $X^1$ | n | A | $X^2$<br>‖<br>$-C-R^3$ | Ar |
|---|---|---|---|---|---|---|
| $NO_2$ | H | O | 1 | $-CH_2-$ | O<br>‖<br>$-C-C_2H_5$ | 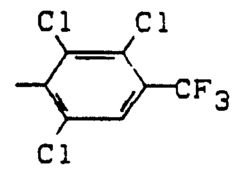 |
| $NO_2$ | H | O | 1 | $CH_3$<br>\|<br>$-CH-$ | S<br>‖<br>$N-NH-C-N(CH_3)_2$<br>‖<br>$-C-H$ | 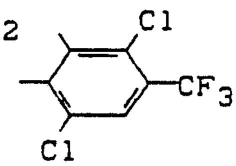 |
| $NO_2$ | H | O | 1 | $-CH_2-$ | $N-NH_2$<br>‖<br>$-C-H$ | 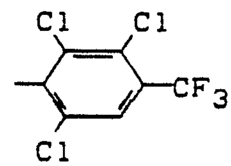 |
| $NO_2$ | $CH_3$ | O | 0 | - | $N-NH_2$<br>‖<br>$-C-CH_3$ | 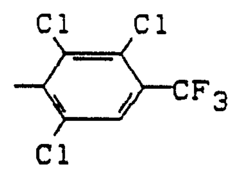 |
| $NO_2$ | $CH_3$ | O | 0 | - | O<br>‖<br>$-C-H$ | 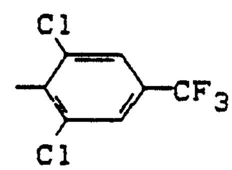 |
| $NO_2$ | $CH_3$ | O | 0 | - | $N-OCH_3$<br>‖<br>$-C-CH_3$ | 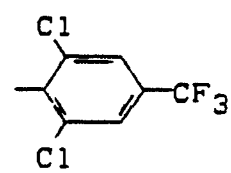 |
| $NO_2$ | $CH_3$ | O | 1 | $-CH_2-$ | $N-OC_2H_5$<br>‖<br>$-C-H$ | 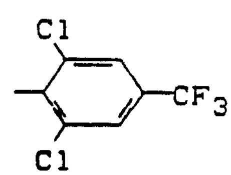 |

Verwendet man beispielsweise 5-Amino-1-(2,4-dichlorphenyl)-pyrazol und α-Methoximinophenylessigsäure-chlorid als Ausgangsstoffe, so läßt sich der Reaktionsablauf des erfindungsgemäßen Verfahrens (a) durch das folgende Formelschema darstellen:

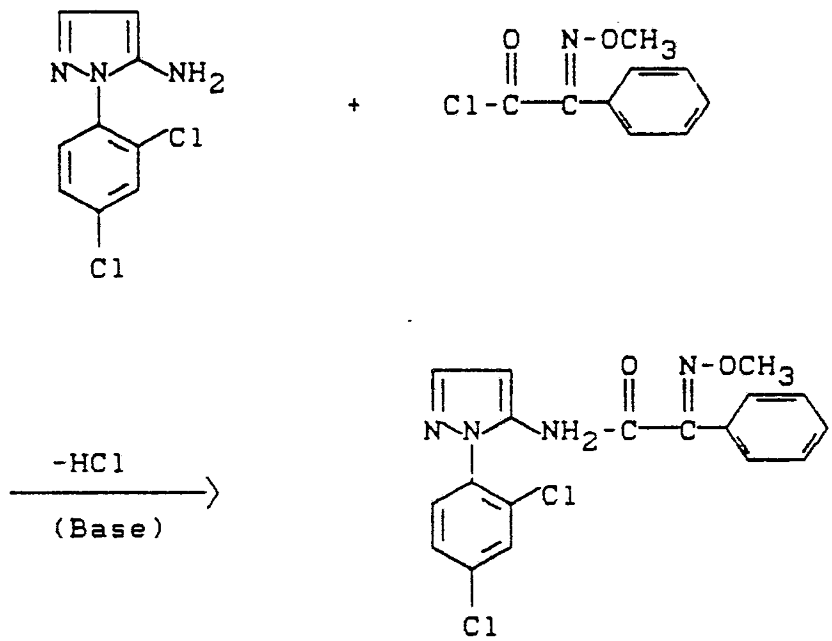


Verwendet man beispielsweise 1-(4-Chlorphenyl)-5-methoximino-acetamido-pyrazol und Methyliodid als Ausgangsstoffe, so läßt sich der Reaktionsablauf des erfindungsgemäßen Verfahrens (b) durch das folgende Formelschema darstellen:

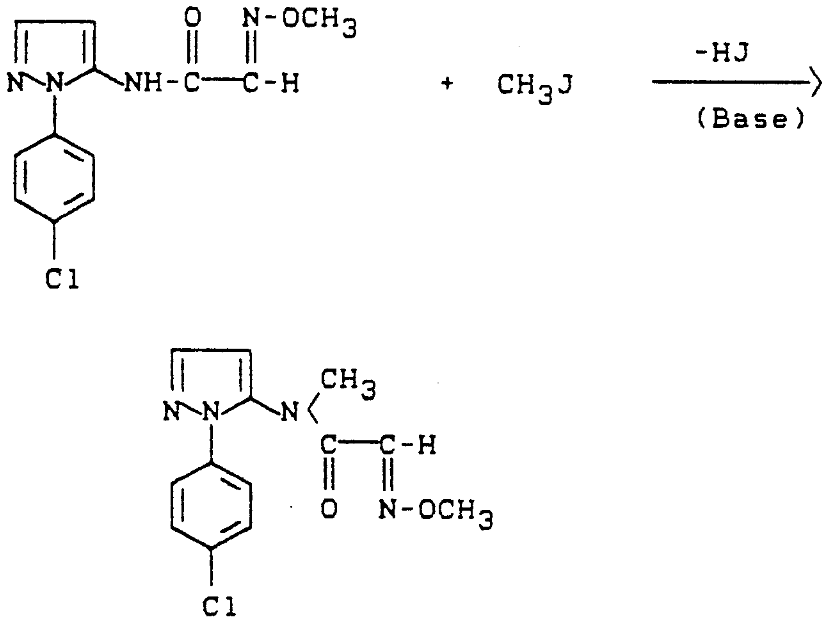


Verwendet man beispielsweise 5-Acetoacetylamino-1-(2-chlor-4-trifluormethyl-phenyl)-4-nitro-pyrazol und N,N-Dimethylhydrazin als Ausgangsstoffe, so läßt sich der Reaktionsablauf des erfindungsgemäßen Verfahrens (c) durch das folgende Formelschema darstellen:

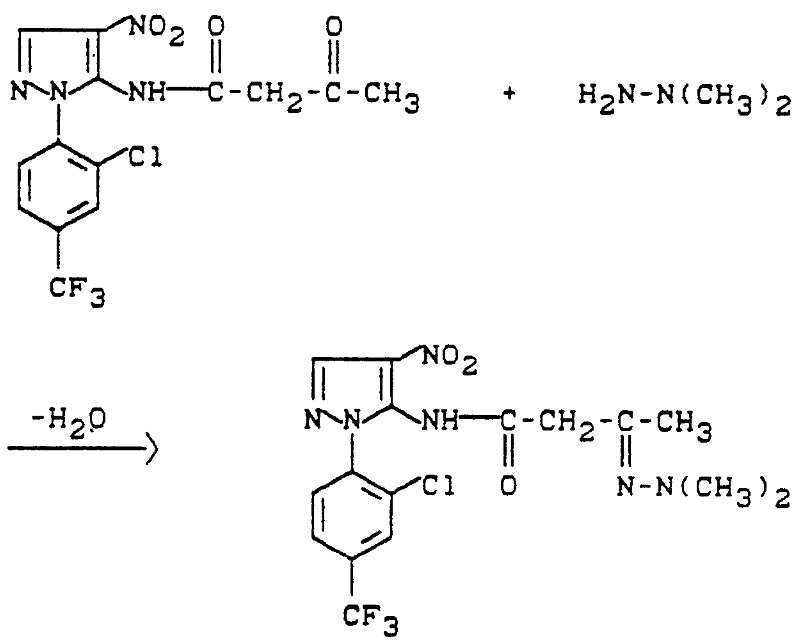

Verwendet man beispielsweise 1-(2,4,6-Trichlorphenyl)-5-methoximinoacetamido-pyrazol und Salpetersäure als Ausgangsstoffe, so läßt sich der Reaktionsablauf des erfindungsgemäßen Verfahrens (d) durch das folgende Formelschema darstellen:

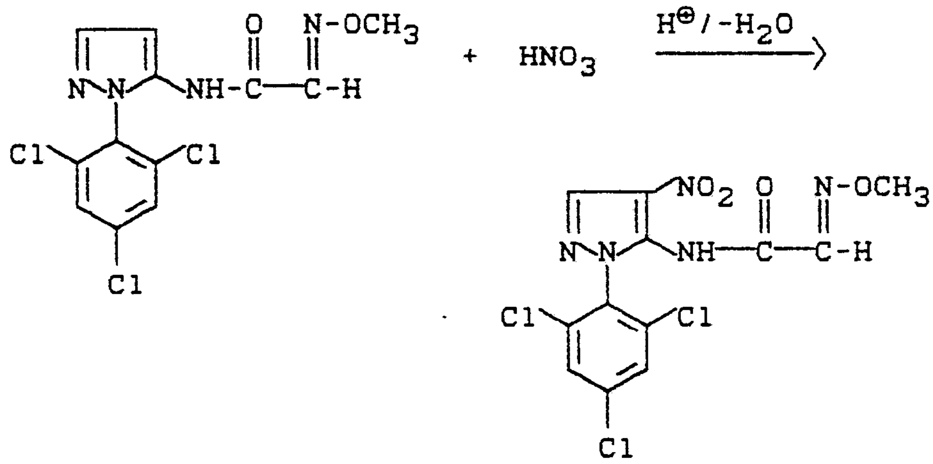


Die zur Durchführung des erfindungsgemäßen Verfahrens (a) als Ausgangsstoffe benötigten 5-Amino-1-aryl-pyrazole sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind teilweise bekannt (vgl. z.B. DE-OS 3 402 308 und DE-OS 3 520 330), teilweise sind sie Gegenstand der eigenen vorgängigen noch nicht publizierten deutschen Patentanmeldung P 35 43 033 vom 05. Dezember 1985 und erhältlich in Analogie zu bekannten Verfahren (vgl. z.B. DE-OS 34 02 308) beispielsweise, indem man Arylhydrazine der Formel (VII),

$$Ar - NH - NH_2 \quad (VII)$$

in welcher

Ar die oben angegebene Bedeutung hat,

mit 2-Halogenacrylnitrilen der Formel (VIII),

$$CH_2{=}C\langle{}^{CN}_{Hal} \quad (VIII)$$

in welcher

Hal für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (IX),

$$Ar - NH - NH - CH_2 - CH\langle{}^{CN}_{Hal} \quad (IX)$$

in welcher

Ar und Hal die oben angegebene Bedeutung haben,

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50 °C und +150 °C cyclisiert,
oder direkt in einem Reaktionsschritt ohne Isolierung der Zwischenstufe der Formel (IX), gegebenenfalls in Gegenwart eines Verdünnungsmittels wie beispielsweise Ethylenglykolmonoethylether oder Ethanol bei Temperaturen zwischen +50 °C und +150 °C cyclisiert und die so erhältlichen 4-unsubstituierten 5-Amino-1-aryl-pyrazole der Formel (IIa),

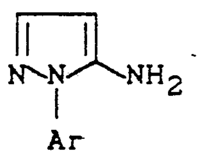

(IIa)

in welcher

Ar die oben angegebene Bedeutung hat,

gegebenenfalls in einer Folgereaktion mit einem Nitrierungsmittel, wie beispielsweise Salpetersäure gegebenenfalls in Gegenwart eines Verdünnungsmittels wie beispielsweise Eisessig und gegebenenfalls in Gegenwart

eines Reaktionshilfsmittels wie beispielsweise Acetanhydrid bei Temperaturen zwischen -20°C und +50°C nitriert oder alternativ mit einem Halogenierungsmittel, wie beispielsweise Chlor, Sulfurylchlorid, Phosphorpentachlorid, N-Chlorsuccinimid, Brom, Phosphortribromid oder N-Bromsuccinimid, gegebenenfalls in Gegenwart eines Verdünnungsmittels, wie beispielsweise Methylenchlorid oder Eisessig, und gegebenenfalls in Gegenwart eines Reaktionshilfsmittels wie beispielsweise Bortrifluorid bei Temperaturen zwischen -20 °C und +50 °C halogeniert.

Dabei kann es gegebenenfalls von Vorteil sein, vor der Halogenierungs- bzw. Nitrierungsreaktion die Aminogruppe in der 5-Position des Pyrazolringes mit Hilfe üblicher Schutzgruppentechnik beispielsweise durch Acylierung zu schützen und die Aminoschutzgruppe nach erfolgter Halogenierung bzw. Nitrierung ebenfalls in üblicher Art und Weise beispielsweise durch Verseifung mit wässriger oder alkoholischer Base wieder abzuspalten.

Die Arylhydrazine der Formel (VII) sind bekannt (vgl. z.B. US-PS 4.127.575; US-PS 3.609.158; DE-OS 2 558 399; J. Chem. Soc. C. 1971, 167-174) oder können nach bekannten Verfahren in einfacher analoger Weise erhalten werden (vgl. z.B. Houben-Weyl "Methoden der organischen Chemie" Band X/2, s. 203, Thieme Verlag Stuttgart 1967; DE-OS 3 402 308).

Die Halogenacrylnitrile der Formel (VIII) sind allgemein bekannte Verbindungen der organischen Chemie.

Die zur Durchführung des erfindungsgemäßen Verfahrens (a) weiterhin als Ausgangsstoffe benötigten Acylierungsmittel sind durch die Formel (III) allgemein definiert. In dieser Formel (III) stehen $X^1$, $X^2$, $R^3$, A und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

$E^1$ steht vorzugsweise für Halogen, insbesondere Chlor oder Brom, oder für einen Rest

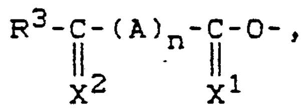

, wobei $X^1$. $X^2$, $R^3$, A und der Index n die oben angegebenen Bedeutungen haben.

Die Acylierungsmittel der Formel (III) sind allgemein bekannte Verbindungen der organischen Chemie oder erhältlich nach allgemein bekannten Verfahren in Analogie zu bekannten Verbindungen [vgl. z.B.: DE-OS 3 208 329; DE-OS 3 208 330; Org. Syntheses <u>61</u>, 1-4 (1983), J. org. Chem. <u>46</u>, 3346 - 3348 (1981); J. chem. Soc. Chem. Commun. 1180 - 1181 (1979)].

Die zur Durchführung des erfindungsgemäßen Verfahrens (b) als Ausgangsstoffe benötigten 5-Acylamino-pyrazol-Derivate sind durch die Formel (Ia) allgemein definiert. In dieser Formel (Ia) stehen $R^1$, $R^3$, $X^1$, $X^2$, A, der Index n und Ar vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (Ia) sind erfindungsgemäße Verbindungen und erhältlich mit Hilfe der erfindungsgemäßen Verfahren (a), (c) oder (d).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für geradkettiges oder verzweigtes Alkyl mit 1 bis 12 Kohlenstoffatomen, für jeweils geradkettiges oder verzweigtes Alkenyl oder Alkinyl mit jeweils 3 bis 8 Kohlenstoffatomen sowie für gegebenenfalls einfach oder mehrfach, gleich oder verschieden durch $C_1$-$C_4$-Alkyl substituiertes Cycloalkyl mit 3 bis 8 Kohlenstoffatomen.

Besonders bevorzugt steht $R^{2-1}$ für geradkettiges oder verzweigtes Alkyl mit 1 bis 8 Kohlenstoffatomen, für jeweils geradkettiges oder verzweigtes Alkenyl oder Alkinyl mit jeweils 3 bis 6 Kohlenstoffatomen sowie für gegebenenfalls einfach bis dreifach, gleich oder verschieden durch Methyl, Ethyl oder Isopropyl substituiertes Cycloalkyl mit 3 bis 6 Kohlenstoffatomen.

Insbesondere steht $R^{2-1}$ für Methyl, Ethyl, Allyl, Propargyl oder Cyclohexyl.

$E^2$ steht vorzugsweise für Chlor, Brom oder Iod, für Methoxysulfonyloxy oder für p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten 5-Acylamino-pyrazole sind durch die Formel (Ie) allgemein definiert. In dieser Formel (Ie) stehen $R^1$, $R^2$, $R^3$, $X^1$, A, Ar und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Acylamino-pyrazole der Formel (Ie) sind erfindungsgemäße Verbindungen und erhältlich mit Hilfe der erfindungsgemäßen Verfahren (a), (b) oder (d).

Die zur Durchführung des erfindungsgemäßen Verfahrens (c) weiterhin als Ausgangsstoffe benötigten primären Aminoverbindungen sind durch die Formel (V) allgemein definiert. In dieser Formel (V) steht Y vorzugsweise für einen Rest $-OR^4$ oder für einen Rest

$$-N\langle{}^{R^5}_{R^6}$$, wobei $R^4$, $R^5$ und $R^6$

vorzugsweise für diejenigen Reste stehen, die bereits im Zusammenhang mit der Beschreibung der erfindungsgemäßen Stoffe der Formel (I) als bevorzugt für diese Substituenten genannt wurden.

Die primären Aminoverbindungen der Formel (V) sind allgemein bekannte Verbindungen der organischen Chemie.

Die zur Durchführung des erfindungsgemäßen Verfahrens (d) als Ausgangsstoffe benötigten 5-Acylamino-pyrazol-Derivate sind durch die Formel (If) allgemein definiert.

In dieser Formel (If) stehen $R^2$, $R^3$, $X^1$, $X^2$, A, Ar und der Index n vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (If) sind erfindungsgemäße Verbindungen und erhältlich mit Hilfe der erfindungsgemäßen Verfahren (a), (b) oder (c).

Die zur Durchführung des erfindungsgemäßen Verfahrens (d) weiterhin als Ausgangsstoffe benötigten Halogenierungs- oder Nitrierungsmittel sind durch die Formel (VI) allgemein definiert. In dieser Formel (VI) steht $R^{1-1}$ vorzugsweise für Chlor, Brom oder Nitro.
$E^3$ steht vorzugsweise für eine übliche Abgangsgruppe, wie beispielsweise Halogen sowie phosphor- oder schwefelhaltige halogenierte Abgangsgruppen. Geeignete Halogenierungs- und Nietrierungsmittel sind beispielsweise Salpetersäure, Nitriersäure, Sulfurylchlorid, Phosphoroxychlorid, Phosphoroxybromid, Phosphortribromid und ähnliche allgemein übliche Halogenierungs- und Nitrierungsmittel.

Die Halogenierungs- und Nitrierungsmittel der Formel (VI) sind allgemein bekannte Verbindungen.

Als Verdünnungsmittel zur Durchführung des erfindungsgemäßen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol,

Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide wie Diemethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Säurebindemittels durchgeführt.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäße Verfahren (a) kann auch gegebenenfalls in Gegenwart eines geeigneten Acylierungskatalysators durchgeführt werden. Als solche verwendet man vorzugsweise Protonensäuren wie Schwefelsäure, Salzsäure, Phosphorsäure, Trifluoressigsäure oder Lewis-Säuren wie Aluminiumtrichlorid, Bortrifluorid oder Eisentrichlorid.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +150 °C, vorzugsweise bei Temperaturen zwischen 0 °C und 100 °C.

Zur Durchführung des erfindungsgemäßen Verfahrens (a) setzt man pro Mol an 5-Amino-1-aryl-pyrazol der Formel (II) im allgemeinen 1.0 bis 15.0 Mol, vorzugsweise 1.0 bis 5.0 Mol an Acylierungsmittel der Formel (III), gegebenenfalls 1.0 bis 3.0 Mol, vorzugsweise 1.0 bis 2.0 Mol an Säurebindemittel oder gegebenenfalls 0.1 bis 3.0 Mol, vorzugsweise 0.1 bis 2.0 Mol an Acylierungskatalysator ein. Es ist auch möglich die Acylierungsmittel der Formel (III) in einer vorgelagerten Reaktion nach allgemein bekannten Verfahren herzustellen und direkt aus dem Reaktionsgemisch heraus im "Eintopfverfahren" gemäß dem erfindungsgemäßen Verfahren (a) weiter umzusetzen (vgl. Herstellungsbeispiele). Die Reaktionsdurchführung, Aufarbeitung und Isolierung der Reaktionsprodukte der Formel (Ia) erfolgt nach üblichen, bekannten Methoden.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel.

Das erfindungsgemäße Verfahren (b) kann gegebenenfalls auch in einem Zweiphasensystem, wie beispielsweise Wasser/Toluol oder Wasser/Dichlormethan, gegebenenfalls

in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natrimhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (b) setzt man pro Mol an 5-Acylamino-pyrazol-Derivate der Formel (Ia) im allgemeinen 1.0 bis 20.0 Mol, vorzugsweise 1.0 bis 15.0 Mol an Alkylierungsmittel der Formel

(IV) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (Ib) erfolgt in allgemein üblicher Art und Weise.

Als Verdünnungsmittel zur Durchführung des erfindungsgemäßen Verfahrens (c) kommen inerte organische Lösungsmittel oder wässrige Systeme infrage. Vorzugsweise verwendet man die bei Verfahren (a) genannten organischen Lösungsmittel. Darüberhinaus bevorzugt sind polare organische Lösungsmittel wie Methanol, Ethanol, n-Propanol, i-Propanol, n-Butanol, i-Butanol, t-Butanol, Ethylenglykol, Diethylenglykol, Ethylenglykolmonomethylether oder -monoethylether, diethylenglykolmonoethylether oder -monoethylether, deren Gemische mit Wasser oder auch reines Wasser als Verdünnungsmittel.

Das erfindungsgemäße Verfahren (c) kann gegebenenfalls in Gegenwart eines geeigneten Reaktionshilfsmittels durchgeführt werden. Als solche verwendet man üblicherweise anorganische oder organische Säuren. Besonders bevorzugt als Reaktionshilfsmittel ist p-Toluolsulfonsäure.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und 100 °C, vorzugsweise bei Temperaturen zwischen 20 °C und 80 °C.

Zur Durchführung des erfindungsgemäßen Verfahrens (c) setzt man pro Mol an 5-Acylamino-pyrazol-Derivat der Formel (Ie) im allgemeinen 1.0 bis 10.0 Mol, vorzugsweise 1.0 bis 5.0 Mol an primärer Aminoverbindung der Formel (V) und gegebenenfalls 0.01 Mol bis 1.0 Mol an Reaktionshilfsmittel ein. Es ist auch möglich die primären Aminoverbindungen der Formel (V) in Form ihrer Säureadditionssalze wie beispielsweise Hydrohalogenide einzusetzen. Dabei kann es gegebenenfalls von Vorteil sein eine äquimolare Menge oder weniger an einer geeigneten Hilfsbase wie beispielsweise Triethylamin oder Pyridin zuzusetzen. Die Reaktionsdurchführung, Aufarbeitung und Isolierung der Reaktionsprodukte der Formel (Ic) erfolgt nach allgemein bekannten Verfahren.

Als Verdünnungsmittel zur Durchführung des Herstellungsverfahrens (d) kommen alle üblicherweise für derartige elektrophile Substitutionen verwendbaren Lösungsmittel infrage. Vorzugsweise verwendet man die als Reagenzien infrage kommenden Säuren oder Gemische, wie beispielsweise Salpetersäure, Nitriersäure, Sulfurylchlorid oder Nitriersäure gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel infrage.

Als Katalysatoren oder Reaktionshilfsmittel zur Durchführung des Herstellungsverfahrens (d) kommen ebenfalls die für derartige Reaktionen üblichen Katalysatoren infrage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (d) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (d) setzt man pro Mol 5-Acylamino-pyrazol-Derivat der Formel (If) im allgemeinen 1.0 bis 10.0 Mol, vorzugsweise 1.0 bis 5.0 Mol an Halogenierungs- oder Nitrierungsmittel der Formel (VI) und gegebenenfalls 0.1 und 10 Mol an Katalysator oder Reaktionshilfsmittel ein. Die Reaktionsführung, Aufarbeitung und Isolierung der Reaktionsprodukte der Formel (Id) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in mono- und dikotylen Kulturen, wie beispielsweise Weizen oder Baumwolle einsetzten.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsions-Konzentrate, wirkstoffimprägnierte Natur- und synthetische Stoffe, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie

Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate,

Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylproyl)-1,3,5-triazin-

2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit 2-Chlor-N-{[(4-methoxy-6-methyl-1,3,5-triazin-2-yl)-amino]-carbonyl}-benzolsulfonamid; N,N-Dimethyl-N'-(3-chlor-4-methylphenyl)-harnstoff; N,N-Dimethyl-N'-(4-isopropylphenyl)-harnstoff; 4-Ethylamino-2-t-butylamino-6-methylthio-s-triazin; 2-Chlor-4-ethylamino-6-isopropylamino-1,3,5-triazin; 2-Chlor-4,6-bis-(ethylamino)-1,3,5-triazin; 2-Chlor-4-ethylamino-6-(3-cyanopropylamino)-1,3,5-triazin; 4-Amino-6-t-butyl-3-ethylthio-1,2,4-triazin-5(4H)-on; 2-[4-(3,5-Dichlorpyrid-2-yloxy)-phenoxy]-propion-säure-(trimethylsilylmethylester); 2-[4-(2,4-Dichlorphenoxy)-phenoxy]-propionsäure, deren Methyl- oder deren Ethylester; 2-{4-[(6-Chlor-2-benzoxazolyl)-oxy]-phenoxy}-propansäure, deren Methyl- oder deren Ethylester; Methyl-5-(2,4-dichlorphenoxy)-2-nitrobenzoat; Methyl-2-[4,5-dihydro-4-methyl-4-(1-methylethyl)-5-oxo-1H-imidazol-2-yl]-4(5)-methylbenzoat; 3,5-Diiod-4-hydroxybenzonitril; 3,5-Dibrom-4-hydroxy-benzonitril; 2,4-Dichlorphenoxyessigsäure; 2,4-Dichlorphenoxypropionsäure; (4-Chlor-2-methylphenoxy)-propionsäure; (2-Methyl-4-chlorphenoxy)-essigsäure; [(4-Amino-3,5-dichlor-6-fluor-2-pyridinyl)-oxy]-essigsäure bzw. -1-methylheptylester; O-(6-Chlor-3-phenyl-pyridazin-4-yl)-S-octyl-thiocarbonat und 3-Isopropyl-2,1,3-benzothiadiazin-4-on-2,2-dioxid sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Streuen.

Die erfindungsgemäßen Wirkstoffe können sowohl vor als auch nach dem Auflaufen der Pflanzen appliziert werden.

Bei Anwendung im Nachauflaufverfahren können die erfindungsgemäßen Verbindungen alleine oder in Kombination mit emulgierbaren Ölen, oberflächenaktiven Substanzen und anderen Additiven ausgebracht werden.

Sie können auch vor der Saat in den Boden eingearbeitet werden.

Die angewandte Wirkstoffmenge kann in einem größeren

Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

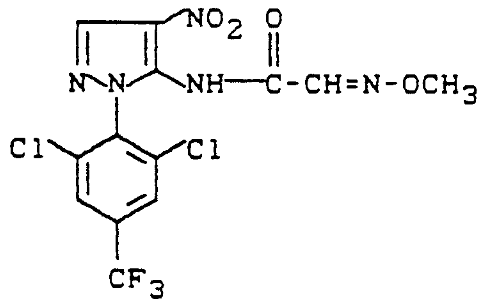

(Verfahren d)

7,6 g (0,02 Mol) 5-Methoximinoacetyl-amino-1-(2,6-dichlor-4-trifluormethyl-phenyl)-pyrazol in 50 ml Eisessig werden bei 10 °C bis 15 °C nacheinander mit 2,5 ml (2,7g/0,045 Mol) Acetanhydrid und dann mit 2,5 ml (2,6 g / 0,06 Mol) 98prozentiger Salpetersäure versetzt. Nach beendeter Zugabe rührt man 3 Stunden bei 20 °C bis 25 °C, gießt dann die Reaktionsmischung in Wasser, extrahiert mit Toluol, wäscht die organische Phase zweimal mit Wasser und einmal mit Natriumhydrogencarbonatlösung, trocknet über Natriumsulfat, engt im Vakuum ein und kristallisiert den Rückstand durch Verreiben mit Ligroin.

Man erhält 4,5 g (53 % der Theorie) an 4-Nitro-5-methoximinoacetyl-amino-1-(2,6-dichlor-4-trifluormethyl-phenyl)-pyrazol vom Schmelzpunkt 107 °C - 109 °C.

Beispiel 2:

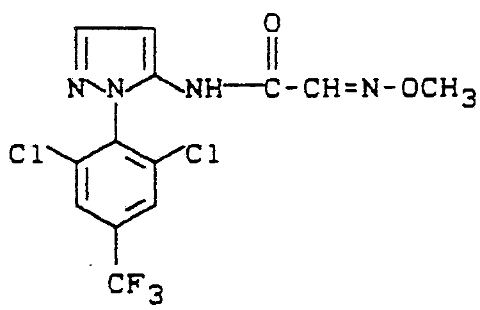

(Verfahren a)

Zu 10,5 g (0,1 Mol) Methoximinoessigsäure (vgl. DE-OS 3 208 329) in 100 ml Acetonitril gibt man zunächst 8 g (o,1 Mol) Pyridin und dann bei 0 °C bis 5 °C tropfenweise 12 g (0,1 Mol) Thionylchlorid. Nach beendeter Zugabe rührt man 3 Stunden bei 30 °C, setzt anschließend 15 g (0,05 Mol) 5-Amino-1-(2,6-dichlor-4-trifluormethyl-phenyl)-pyrazol (vgl. DE-OS 3 402 308) zu und danach weitere 4 g (0,05 Mol) Pyridin. Danach rührt man weitere 12 Stunden bei 20 °C bis 25 °C, gießt dann die Reaktionsmischung in Wasser, saugt den kristallinen Niederschlag ab und trocknet auf Ton.

Man erhält 15,4 g (81 % der Theorie) an 5-Methoximinoacetyl-amino-1-(2,6-dichlor-4-trifluormethyl-phenyl)-pyrazol vom Schmelzpunkt 158 °C - 160 °C.

In entsprechender Weise und gemäß den allgemeinen Angaben zur Herstellung erhält man die folgenden 5-Acylamino-pyrazol-Derivate der allgemeinen Formel (I):

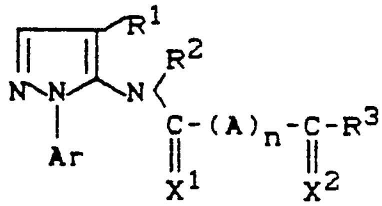

(I)

Tabelle 2:

| Bsp. | $R^1$ | $R^2$ | 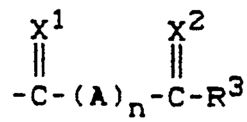 | Ar | Schmelzpunkt/°C |
|---|---|---|---|---|---|
| 3 | H | H | 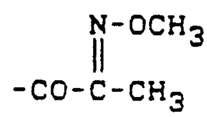 | 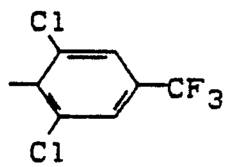 | 96 - 98 |
| 4 | H | H | 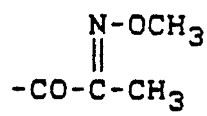 | 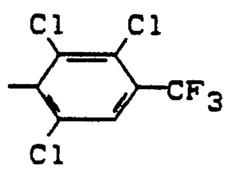 | 91 |
| 5 | $NO_2$ | H | 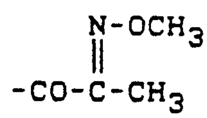 | 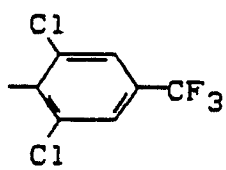 | 150 - 152 |

Tabelle 2 - Fortsetzung

| Bsp. | $R^1$ | $R^2$ | 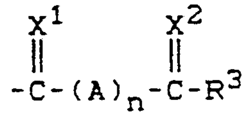 | Ar | Schmelzpunkt/°C |
|---|---|---|---|---|---|
| 6 | $NO_2$ | H | 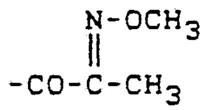 | 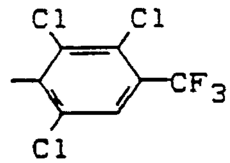 | 143 - 146 |
| 7 | $NO_2$ | H | 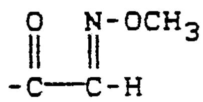 | 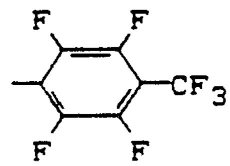 | Öl |
| 8 | H | H | 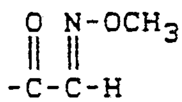 | 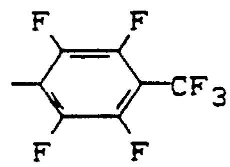 | 108 - 112 |
| 9 | H | H | 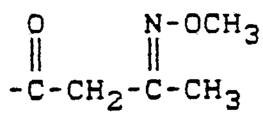 | 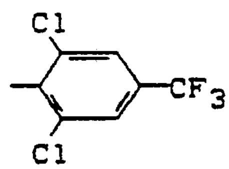 | 104 - 105 |
| 10 | H | H | 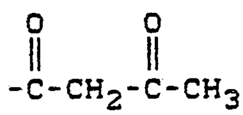 | 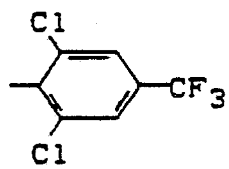 | 73 - 75 |
| 11 | $NO_2$ | H | 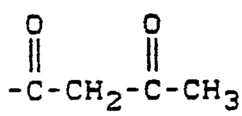 | 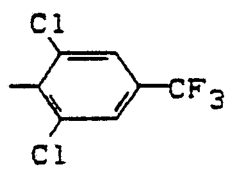 | 64 - 84 |

Tabelle 2 - Fortsetzung

| Bsp. | $R^1$ | $R^2$ | 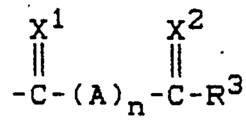 | Ar | Schmelzpunkt/°C |
|---|---|---|---|---|---|
| 12 | $NO_2$ | H | 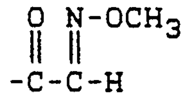 | 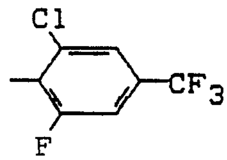 | 88-93 |
| 13 | $NO_2$ | H | 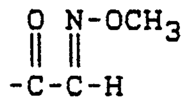 | 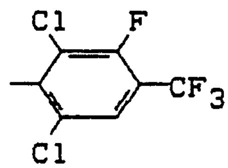 | 113-118 |
| 14 | $NO_2$ | H | 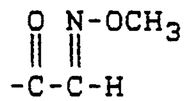 | 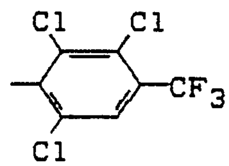 | 147-152 |
| 15 | $NO_2$ | H | 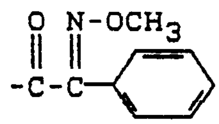 | 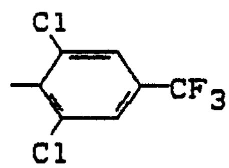 | 90 |
| 16 | $NO_2$ | H | 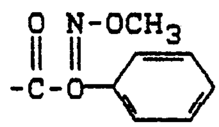 | 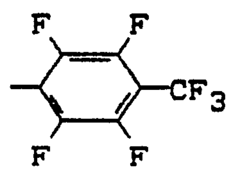 | Öl |
| 17 | $NO_2$ | H | 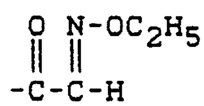 | 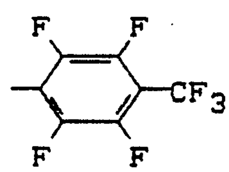 | 100-102 |

Tabelle 2 - Fortsetzung

| Bsp. | $R^1$ | $R^2$ | 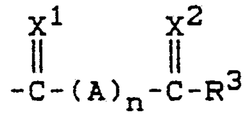 | Ar | Schmelzpunkt/°C |
|---|---|---|---|---|---|
| 18 | $NO_2$ | H | 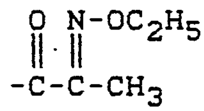 | 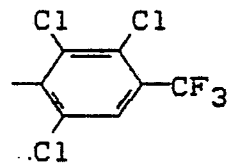 | 129-133 |
| 19 | $NO_2$ | H | 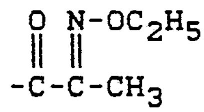 | 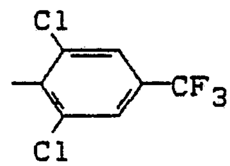 | 114-120 |
| 20 | $NO_2$ | H | 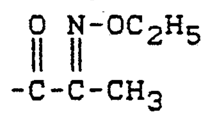 | 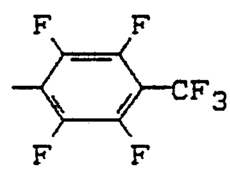 | 40-43 |
| 21 | $NO_2$ | H | 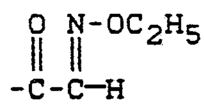 | 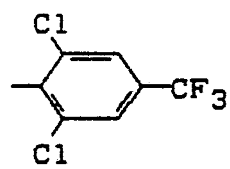 | 127-129 |
| 22 | $NO_2$ | H | 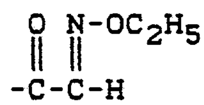 | 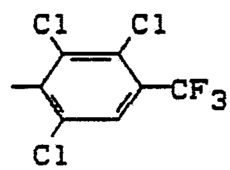 | 163-171 |
| 23 | $NO_2$ | H | 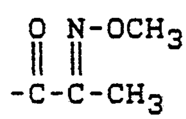 | 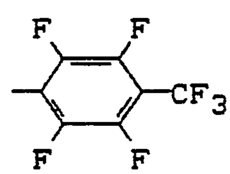 | 134-138 |

Tabelle 2 - Fortsetzung

| Bsp. | $R^1$ | $R^2$ | 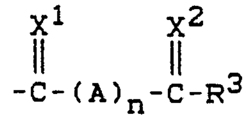 | Ar | Schmelzpunkt/°C |
|---|---|---|---|---|---|
| 24 | $NO_2$ | H | 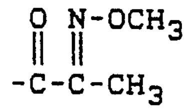 | 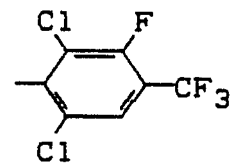 | 138-140 |
| 25 | $NO_2$ | H | 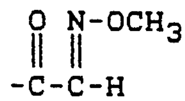 | 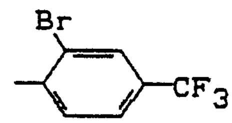 | Oil |
| 26 | $NO_2$ | H | 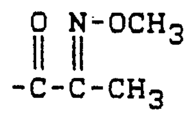 | 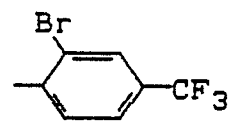 | 145-149 |
| 27 | $NO_2$ | H | 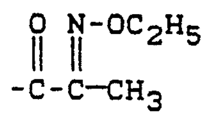 | 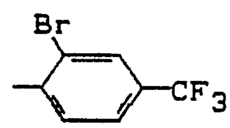 | 92-94 |
| 28 | $NO_2$ | H | 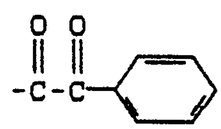 | 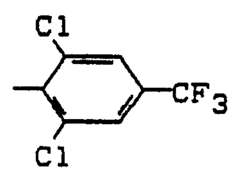 | 142-150 |
| 29 | $NO_2$ | H | 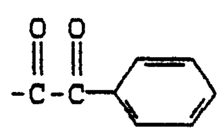 | 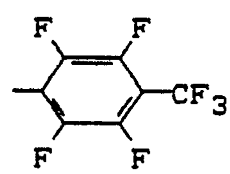 | 129-133 |

Anwendungsbeispiele:

In den folgenden Anwendungsbeispielen wurde die nachstehend aufgeführte Verbindung als Vergleichsubstanz eingesetzt:

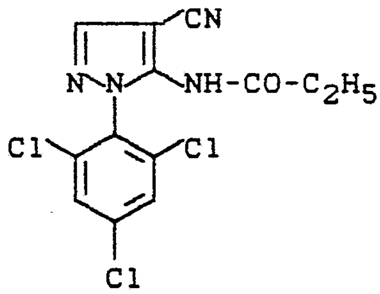

(A)

4-Cyano-5-propionamido-1-(2,4,6-trichlorphenyl)-pyrazol

(bekannt aus DE-OS 3 226 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegen Unkräuter wie beispielsweise Galium, Stellaria und Portulaca ebenso wie in der Nutzpflanzenselektivität wie beispielsweise bei Weizen gegenüber der Vergleichssubstanz (A) zeigen in diesem Test z.B. die Verbindungen gemäß den Herstellungsbeispielen 1, 5, 6, 7, 17, 20, 21, 22, 23 und 24.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

Eine deutliche Überlegenheit in der Wirksamkeit gegen Unkräuter wie beispielsweise Cassia, Chenopodium und Portulaca ebenso wie in der Nutzpflanzenselektivität wie beispielsweise bei Weizen gegenüber der Vergleichssubstanz (A) zeigen in diesem Test z.B. die Verbindungen gemäß den Herstellungsbeispielen: 1, 5, 6, 7, 12, 13, 14, 16, 17, 18, 19, 20, 21, 22, 23, 24, 25, 26 und 27.

## Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen beispielsweise die Verbindungen gemäß den Herstellungsbeispielen 1, 5 und 6 eine deutliche Wirksamkeit im Vergleich zur unbehandelten Kontrolle.

Patentansprüche

1. 5-Acylamino-pyrazol-Derivate der Formel (I),

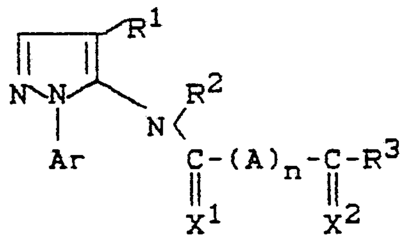

(I)

in welcher

$R^1$ für Wasserstoff, Halogen oder Nitro steht,

$R^2$ für Wasserstoff, Alkyl, Alkenyl, Alkinyl oder gegebenenfall substituiertes Cycloalkyl steht,

$R^3$ für Wasserstoff, Alkyl, für gegebenenfalls substituiertes Cycloalkyl oder für gegebenenfalls substituiertes Aryl steht,

Ar für jeweils gegebenenfalls substituiertes Phenyl oder Pyridyl steht,

$X^1$ für Sauerstoff oder Schwefel steht,

A für eine geradkettige oder verzweigte, gegebenenfalls substituierte Alkylenbrücke steht,

n für die Zahlen 0 oder 1 steht und

$X^2$ für Sauerstoff, für einen Rest $=N-O-R^4$ oder für einen Rest $=N-N\langle{}^{R^5}_{R^6}$ steht,

wobei

$R^4$ für Wasserstoff, Alkyl, Alkenyl, Alkinyl, Halogenalkyl, Halogenalkenyl, Alkoxyalkyl, Alkylthioalkyl, für gegebenenfalls substituiertes Aralkyl oder für gegebenenfalls substituiertes Aryl steht,

$R^5$ für Wasserstoff oder Alkyl steht und

$R^6$ für Wasserstoff, Alkyl, für gegebenenfalls substituiertes Aryl oder für einen Rest

$$-\underset{\underset{X^3}{\|}}{C}-R^7$$ steht,

wobei

$X^3$ für Sauerstoff oder Schwefel steht und

$R^7$ für Wasserstoff, Alkyl, Alkoxy, Alkylamino oder Dialkylamino steht.

2. 5-Acylamino-pyrazol-Derivate der Formel (I), gemäß Anspruch 1, in welcher

$R^1$ für Wasserstoff, Nitro, Fluor, Chlor, Brom oder Iod steht,

$R^2$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 3 bis 8 Kohlenstoffatomen sowie für gegebenenfalls einfach oder mehrfach gleich oder verschieden durch $C_1$-$C_4$-Alkyl substituiertes Cycloalkyl mit 3 bis 8 Kohlenstoffatomen steht,

$R^3$ für Wasserstoff, für geradkettiges oder verzweigtes Alkyl mit 1 bis 8 Kohlenstoffatomen, für gegebenenfalls einfach oder mehrfach, gleich oder verschieden durch $C_1$-$C_4$-Alkyl substituiertes Cycloalkyl mit 3 bis 8 Kohlenstoffatomen oder für gegebenenfalls einfach oder mehrfach, gleich oder verschieden substituiertes Aryl mit 6 bis 10 Kohlenstoffatomen steht, wobei als Arylsubstituenten in Frage kommen: Halogen, Cyano, Nitro sowie jeweils geradkettiges oder verzweigtes Alkyl oder Alkoxy mit jeweils 1 bis 4 Kohlenstoffatomen,

Ar für jeweils gegebenenfalls einfach oder mehrfach gleich oder verschieden substituiertes Phenyl, 2-Pyridyl, 3-Pyridyl oder 4-Pyridyl steht, wobei als Substituenten jeweils in

Frage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m - R^8$,

wobei

$R^8$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für eine Zahl 0, 1 oder 2 steht,

$X^1$ für Sauerstoff oder Schwefel steht,

A für eine geradkettige oder verzweigte Alkylenbrücke mit 1 bis 6 Kohlenstoffatomen steht, welche gegebenenfalls einfach oder mehrfach, gleich oder verschieden durch Phenyl, das gegebenenfalls einfach oder mehrfach, gleich oder verschieden durch Halogen oder $C_1$-$C_4$-Alkyl substituiert ist, oder durch Halogen substituiert ist,

n für eine Zahl 0 oder 1 steht und

$X^2$ für Sauerstoff, für einen Rest $=N-O-R^4$ oder für einen Rest $=N-N\langle^{R^5}_{R^6}$ steht, wobei

$R^4$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 8 Kohlenstoffatomen, für jeweils geradkettiges oder verzweigtes Halogenalkyl mit 1 bis 8 Kohlenstoffatomen oder Halogenalkenyl mit 3 bis 8 Kohlenstoffatomen und jeweils 1 bis 9 gleichen oder verschiedenene Halogenatomen, für jeweils geradkettiges oder verzweigtes Alkoxyalkyl oder Alkylthioalkyl mit jeweils 1 bis 4 Kohlenstoffatomen in den einzelnen Alkylteilen oder für jeweils im Arylteil einfach oder mehrfach, gleich oder verschieden substituiertes Aralkyl mit 1 bis 4 Kohlenstoffatomen im Alkylteil oder Aryl mit jeweils 6 bis 10 Kohlenstoffatomen im Arylteil steht, wobei als Arylsubstituenten jeweils in Frage kommen: Halogen, Cyano, Nitro, sowie jeweils geradkettiges oder verzweigtes Alkyl oder Alkoxy mit jeweils 1 bis 4 Kohlenstoffatomen,

$R^5$ für Wasserstoff oder für geradkettiges oder verzweigtes Alkyl mit 1 bis 6 Kohlenstoffatomen steht und

$R^6$ für Wasserstoff, für geradkettiges oder verzweigtes Alkyl mit 1 bis 6 Kohlenstoffatomen, für gegebenenfalls einfach oder mehrfach, gleich oder verschieden substituiertes Aryl mit 6 bis 10 Kohlenstoffatomen oder für einen Rest $-\underset{\underset{X^3}{\|}}{C}-R^7$ steht,

wobei als Arylsubstituenten die bei $R^4$ genannten infrage kommen,

$X^3$ für Sauerstoff und Schwefel steht und

$R^7$ für Wasserstoff oder für jeweils geradkettiges oder verzweigtes Alkyl, Alkoxy, Alkylamino oder Dialkylamino mit jeweils 1 bis 8 Kohlenstoffatomen in den einzelnen Alkylteilen steht.

3. 5-Acylamino-pyrazol-Derivate der Formel (I), gemäß Anspruch 1, in welcher

$R^1$ für Wasserstoff, Nitro, Chlor oder Brom steht,

$R^2$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 6 Kohlenstoffatomen sowie für gegebenenfalls einfach bis dreifach, gleich oder verschieden durch Methyl, Ethyl oder Isopropyl substituiertes Cycloalkyl mit 3 bis 6 Kohlenstoffatomen steht,

$R^3$ für Wasserstoff, für geradkettiges oder verzweigtes Alkyl mit 1 bis 6 Kohlenstoffatomen, für gegebenenfalls einfach bis dreifach, gleich oder verschieden durch Methyl, Ethyl oder Isopropyl substituiertes Cycloalkyl mit 3 bis 6 Kohlenstoffatomen oder für gegebenenfalls ein- bis fünffach, gleich oder verschieden substituiertes Phenyl steht, wobei als Phenylsubstituenten infrage kommen: Fluor, Chlor, Brom, Iod, Cyano, Nitro, Methyl, Ethyl, n- oder i-Propyl, n-, i-, s- oder t-Butyl, Methoxy oder Ethoxy,

Ar für gegebenenfalls ein- bis fünffach, gleich oder verschieden substituiertes Phenyl oder für jeweils gegebenenfalls ein- bis vierfach, gleich oder verschieden substituiertes 2-Pyridyl, 3-Pyridyl oder 4-Pyridyl steht, wobei als Phenyl- und Pyridyl-Substituenten jeweils in Frage kommen: Cyano, Nitro, Fluor, Chlor, Brom, Iod, Methyl, Ethyl, n- oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^8$,

wobei

$R^8$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und

m für eine Zahl 0, 1 oder 2 steht,

$X^1$ für Sauerstoff oder Schwefel steht,

A für ein Brückenglied der Formel $-CH_2-$;

$$-CH_2-CH_2-;\ -\underset{\textstyle CH_3}{\underset{|}{CH}}-;\ -CCl_2-CH_2-;\ -CHCl-CH_2-,$$

$$-\underset{\textstyle CH_3}{\underset{|}{CH}}-CH_2-;\ -CH_2-CH_2-CH_2-;\ -CH_2-CH_2-CH_2-CH_2-$$

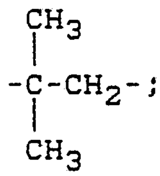 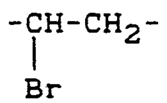 $-CBr_2-CH_2-$; $-C(CH_3)_2-$;

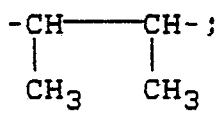 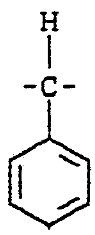 oder 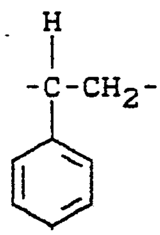 steht;

n für eine Zahl 0 oder 1 steht und

$X^2$ für Sauerstoff, für einen Rest $=N-O-R^4$ oder für einen Rest

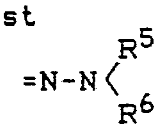 steht,

wobei

$R^4$ für Wasserstoff, für geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen, für jeweils geradkettiges oder verzweigtes Halogenalkyl mit 1 bis 6 Kohlenstoffatomen oder Halogenalkenyl mit 3 bis 6 Kohlenstoffatomen und jeweils 1 bis 5 gleichen oder verschiedenen Halogenatomen,

für jeweils geradkettiges oder verzweigtes Alkoxyalkyl oder Alkylthioalkyl mit jeweils 1 bis 3 Kohlenstoffatomen in den einzelnen Alkylteilen oder für jeweils im Phenylteil ein- bis fünffach, gleich oder verschieden substituiertes Benzyl, Phenylethyl oder Phenyl steht, wobei als Phenylsubstituenten jeweils infrage kommen: Fluor, Chlor, Brom, Iod, Cyano, Nitro, Methyl, Ethyl, n- oder i-Propyl, n-, i-, s- oder t-Butyl, Methoxy oder Ethoxy,

$R^5$ für Wasserstoff oder für geradkettiges oder verzweigtes Alkyl mit 1 bis 4 Kohlenstoffatomen steht und

$R^6$ für Wasserstoff, für geradkettiges oder verzweigtes Alkyl mit 1 bis 4 Kohlenstoffatomen, für gegebenenfalls ein- bis fünffach, gleich oder verschieden substituiertes Phenyl oder für einen Rest

$$-\underset{\underset{X^3}{\|}}{C}-R^7 \quad \text{steht,}$$

wobei als Phenylsubstituenten die bei $R^4$ genannten infrage kommen,

$X^3$ für Sauerstoff oder Schwefel steht und

$R^7$ für Wasserstoff, für geradkettiges oder verzweigtes Alkyl mit 1 bis 6 Kohlenstoffatomen oder für jeweils geradkettiges oder verzweigtes Alkoxy, Alkylamino oder Dialkylamino mit jeweils 1 bis 4 Kohlenstoffatomen in den einzelnen Alkylteilen steht.

4. Verfahren zur Herstellung von 5-Acylamino-pyrazol-Derivaten der Formel (I),

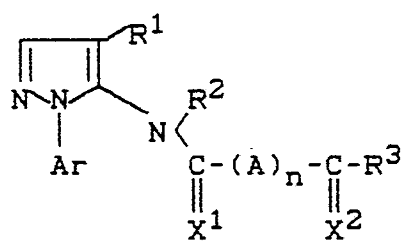

(I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff, Alkyl, für gegebenenfalls substituiertes Cycloalkyl oder für gegebenenfalls substituiertes Aryl steht,

Ar für jeweils gegebenenfalls substituiertes Phenyl oder Pyridyl steht,

$X^1$ für Sauerstoff oder Schwefel steht,

A für eine geradkettige oder verzweigte, gegebenenfalls substituierte Alkylenbrücke steht,

n für die Zahlen 0 oder 1 steht und

$X^2$ für Sauerstoff, für einen Rest $=N-O-R^4$ oder für

einen Rest $=N-N\langle{}^{R^5}_{R^6}$ steht,

wobei

$R^4$ für Wasserstoff, Alkyl, Alkenyl, Alkinyl, Halogenalkyl, Halogenalkenyl, Alkoxyalkyl, Alkylthioalkyl, für gegebenenfalls substituiertes Aralkyl oder für gegebenenfalls substituiertes Aryl steht,

$R^5$ für Wasserstoff oder Alkyl steht und

$R^6$ für Wasserstoff, Alkyl, für gegebenenfalls substituiertes Aryl oder für einen Rest

$$-\underset{\underset{X^3}{\|}}{C}-R^7$$ steht.

wobei

$X^3$ für Sauerstoff oder Schwefel steht und

$R^7$ für Wasserstoff, Alkyl, Alkoxy, Alkylamino oder Dialkylamino steht,

dadurch gekennzeichnet, daß man

a) 5-Acylamino-pyrazol-Derivate der Formel (Ia) erhält,

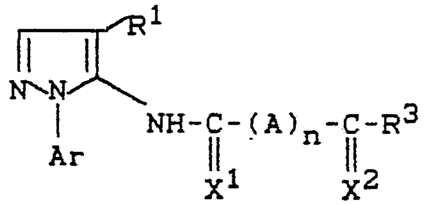

(Ia)

in welcher

$R^1$, $R^3$, $X^1$, $X^2$, A, Ar und der Index n die oben angegebene Bedeutung haben,

wenn man 5-Amino-1-aryl-pyrazole der Formel (II),

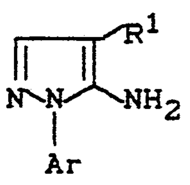

(II)

in welcher

$R^1$ und Ar die oben angegebene Bedeutung haben,

mit Acylierungsmitteln der Formel (III),

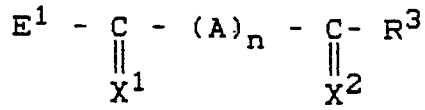

(III)

in welcher

$X^1$, $X^2$, $R^3$, A und der Index n die oben angegebene Bedeutung haben und

$E^1$ für eine elektronenanziehende Abgangsgruppe steht,

gegebenenfalls in Gegenwart eines Verdünnungsmittels, gegebenenfalls in Gegenwart eines Säurebindemittels sowie gegebenenfalls in Gegenwart eines Katalysators umsetzt;

oder daß man

b) 5-Acylamino-pyrazol-Derivate der Formel (Ib) erhält,

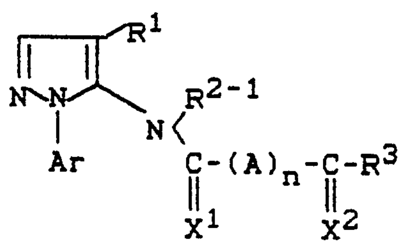

(Ib)

in welcher

$R^1$, $R^3$, $X^1$, $X^2$, A , der Index n und Ar die oben angegebene Bedeutung haben und

$R^{2-1}$ für Alkyl, Alkenyl, Alkinyl oder gegebenenfalls substituiertes Cycloalkyl steht,

wenn man 5-Acylamino-pyrazol-Derivate der Formel (Ia),

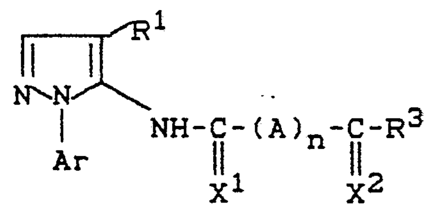

(Ia)

in welcher

$R^1$, $R^3$, $X^1$, $X^2$, A, Ar und der Index n die oben angegebene Bedeutung haben,

mit Alkylierungsmitteln der Formel (IV),

$$E^2 - R^{2-1} \quad (IV)$$

in welcher

$R^{2-1}$ die oben angegebene Bedeutung hat und

$E^2$ für Halogen, für gegebenenfalls substituiertes Alkoxysulfonyloxy oder für gegebenenfalls substituiertes Arylsulfonyloxy steht,

gegebenenfalls in Gegenwart eines Verdünnungsmittels und gegebenenfalls in Gegenwart eines Säurebindemittels sowie gegebenenfalls in Gegenwart eines Katalysators umsetzt;

oder daß man

c) 5-Acylamino-pyrazol-Derivate der Formel (Ic) erhält,

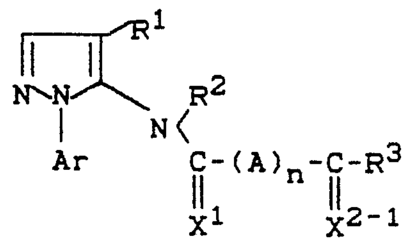

(Ic)

in welcher

$R^1$, $R^2$, $R^3$, $X^1$, A, Ar und der Index n die oben angegebene Bedeutung haben und

$X^{2-1}$ für einen Rest $=N-O-R^4$ oder für einen Rest

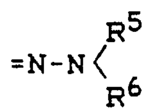

steht,

wobei

$R^4$, $R^5$ und $R^6$ die oben angegebene Bedeutung haben,

wenn man 5-Acylamino-pyrazol-Derivate der Formel (Ie),

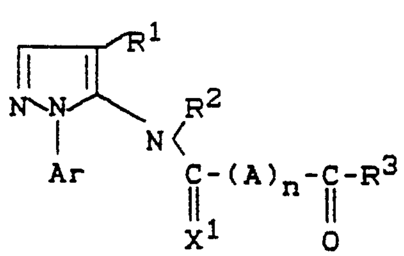

(Ie)

in welcher

$R^1$, $R^2$, $R^3$, $X^1$, A, Ar und der Index n die oben angegebene Bedeutung haben,

mit primären Aminoverbindungen der Formel (V),

$$H_2N - Y \qquad (V)$$

in welcher

Y für einen Rest $-OR^4$ oder für einen Rest $-N\langle{}^{R^5}_{R^6}$ steht, wobei $R^4$, $R^5$ und $R^6$ die oben angegebene Bedeutung haben,

gegebenenfalls in Gegenwart eines Verdünnungsmittels und gegebenenfalls in Gegenwart eines Reaktionshilfsmittels umsetzt;

oder daß man

d) 5-Acylamino-pyrazol-Derivate der Formel (Id) erhält,

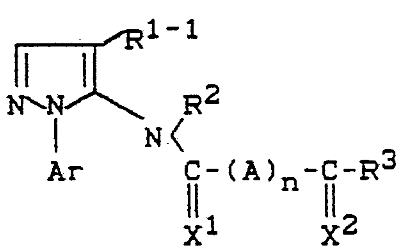

(Id)

in welcher

$R^2$, $R^3$, $X^1$, $X^2$, A, Ar und der Index n die oben angegebene Bedeutung haben und

$R^{1-1}$ für Halogen oder Nitro steht,

wenn man 5-Acylamino-pyrazol-Derivate der Formel (If),

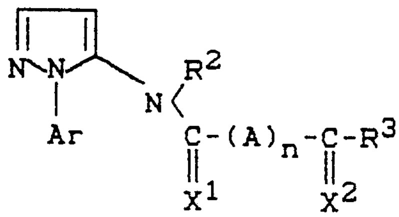

(If)

in welcher

$R^2$, $R^3$, $X^1$, $X^2$, A, Ar und der Index n die oben angegebene Bedeutung haben,

mit Halogenierungs- oder Nitrierungsmitteln der Formel (VI),

$$R^{1-1} - E^3 \quad (VI)$$

in welcher

$R^{1-1}$ die oben angegebene Bedeutung hat und

$E^3$ für eine elektronenanziehnde Abgangsgruppe steht,

gegebenenfalls in Gegenwart eines Verdünnungsmittels und gegebenenfalls in Gegenwart eines Katalysators oder Reaktionshilfsmittels umsetzt.

5. Herbizide und pflanzenwuchsregulierende Mittel, gekennzeichnet durch einen Gehalt an mindestens einem 5-Acylamino-pyrazol-Derivat der Formel (I) gemäß den Ansprüchen 1 bis 4.

6. Verfahren zur Bekämpfung von Unkräutern, dadurch gekennzeichnet, daß man 5-Acylamino-pyrazol-Derivate der Formel (I) gemäß den Ansprüchen 1 bis 4 auf die Unkräuter und/oder ihren Lebensraum einwirken läßt.

7. Verwendung von 5-Acylamino-pyrazol-Derivaten der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man 5-Acylamino-pyrazol-Derivate der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 87 11 5569

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 61, Nr. 13, 21. Dezember 1964, Spalte 16061 f-h, Columbus, Ohio, US; S.V. TABAK et al.: "Pyrazoles. XLII. Condensation of isomeric 1-phenyl-x-aminopyrazoles with beta-dicarbonyl compounds", & ZH. OBSHCH. KHIM. 1964, 34(8), 2786-2759 | 1-4 | C 07 D 231/40<br>C 07 D 401/04<br>A 01 N 43/56 |
| A | EP-A-0 154 115 (BAYER AG)<br>* Ansprüche 1-4 * & DE - A - 3 402 308 (Kat. D) | 1,5-8 | |
| P,A | EP-A-0 207 285 (BAYER AG)<br>* Ansprüche 1, 5-8 * & DE - A - 3 520 330 (Kat. D) | 1,5-8 | |
| | | | RECHERCHIERTE SACHGEBIETE (Int. Cl.4) |
| | | | C 07 D 231/00<br>C 07 D 401/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 01-02-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument